(19) Europäisches Patentamt
European Patent Office
Office européen des brevets

(11) Veröffentlichungsnummer : **0 105 288**
**B1**

(12) **EUROPÄISCHE PATENTSCHRIFT**

(45) Veröffentlichungstag der Patentschrift : **11.12.85**

(21) Anmeldenummer : **83900905.7**

(22) Anmeldetag : **11.03.83**

(86) Internationale Anmeldenummer : **PCT/DE 83/00045**

(87) Internationale Veröffentlichungsnummer : **WO/8303248 (29.09.83 Gazette 83/23)**

(51) Int. Cl.⁴ : **C 07 C177/00**, C 07 C 59/50, C 07 C 69/73, C 07 C 33/12, C 07 C103/737, C 07 D263/14, A 61 K 31/557

(54) **CARBACYCLINE, HERSTELLUNG UND VERWENDUNG.**

(30) Priorität : **12.03.82 DE 3209702**
**18.02.83 DE 3306125**

(43) Veröffentlichungstag der Anmeldung : **18.04.84 Patentblatt 84/16**

(45) Bekanntmachung des Hinweises auf die Patenterteilung : **11.12.85 Patentblatt 85/50**

(84) Benannte Vertragsstaaten : **AT BE CH DE FR GB LI LU NL SE**

(56) Entgegenhaltungen :
**GB-A- 2 013 661**
**US-A- 4 045 468**
**Prostaglandins, Vol. 22, No. 5, November 1981, Los Altos (Calif. US), N.H. Andersen et al.: "Omega chain methylated analogs of PGF2x and PGE2", pages 809-830, see page 810, formulae IVF and VF**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **VORBRÜGGEN, Helmut**
**Wilkestrasse 7**
**D-1000 Berlin 27 (DE)**
Erfinder : **RADÜCHEL, Bernd**
**Gollanczstrasse 132**
**D-1000 Berlin 28 (DE)**
Erfinder : **SKUBALLA, Werner**
**Olwenstrasse 25**
**D-1000 Berlin 28 (DE)**
Erfinder : **SCHWARZ, Norbert**
**Stubenrauchstrasse 31**
**D-1000 Berlin 41 (DE)**
Erfinder : **CASALS-STENZEL, Jorge**
**Sertoriusring 295**
**D-6500 Mainz 21 Finthen (DE)**
Erfinder : **VARCHMIN, Gerda**
**Sachsenring 22-24**
**D-5000 Köln 1 (DE)**
Erfinder : **TOWN, Michael-Harold**
**Zugspitzstrasse 13**
**D-8127 Iffeldorf (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neue Prostacyclin-Derivate, Verfahren zu ihrer Herstellung sowie ihre Verwendung als Arzneimittel.

In den deutschen Offenlegungsschriften DE-OS 28 45 770, 29 00 352, 29 02 442, 29 04 655, 29 09 088 und 29 12 409 werden (5E)- und (5Z)-6a-Carbaprostaglandin-$I_2$-Analoga beschrieben. Die Nomenklatur der erfindungsgemässen Verbindungen basiert auf einem Vorschlag von Morton und Brokaw (J. Org. Chem. *44*, 2880 (1979)). Bei der Synthese dieser Verbindungen entstehen stets zwei Doppelbindungsisomere, die durch den Zusatz (5E) oder (5Z) charakterisiert werden. Die beiden Isomeren dieses Prototyps werden durch folgende Strukturformeln verdeutlicht :

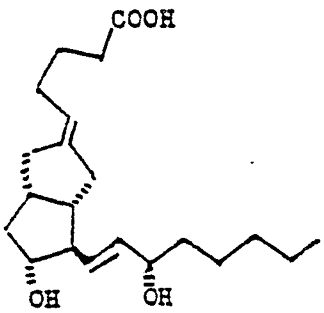

(5E)-6a-Carbaprostaglandin-$I_2$

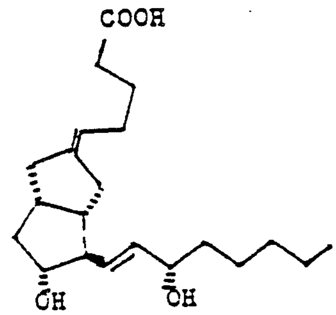

(5Z)-6a-Carbaprostaglandin-$I_2$

Aus dem sehr umfangreichen Stand der Technik der Prostacycline und ihrer Analoga weiss man, dass diese Stoffklasse auf Grund ihrer biologischen und pharmakologischen Eigenschaften zur Behandlung von Säugetieren, einschliesslich Menschen geeignet ist. Ihre Verwendung als Arzneimittel stösst jedoch häufig auf Schwierigkeiten, da sie eine für therapeutische Zwecke zu kurze Wirkungsdauer besitzen. Alle Strukturveränderungen haben das Ziel, die Wirkungsdauer sowie die Selektivität der Wirksamkeit zu steigern.

Es wurde nun gefunden, daß durch Einführung einer Cycloalkylgruppe in die 16-Position des Carbycyclins eine längere Wirkungsdauer, größere Selektivität und bessere Wirksamkeit erzielt werden kann. Die erfindungsgemäßen Verbindungen wirken blutdrucksenkend und bronchodilatatorisch. Sie sind außerdem zur Inhibierung der Thrombozytenaggregation, der Vasodilatation und der Inhibierung der Magensäuresekretion geeignet.

Die Erfindung betrifft Carbacyclinderivate der allgemeinen Formel I

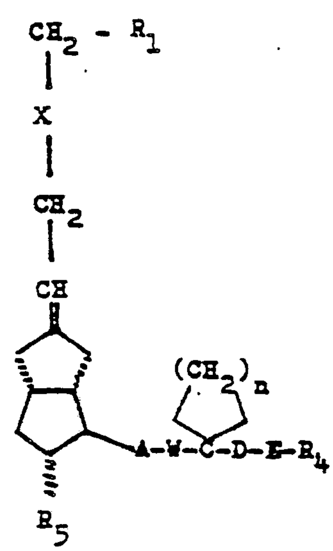

(I)

worin

$R_1$ den Rest $CH_2OH$ oder

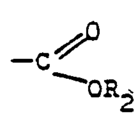

mit $R_2$ in der Bedeutung eines Wasserstoffatoms, eines Alkyl-, Cycloalkyl, Aryl, eines

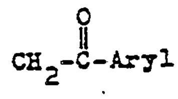

oder heterocyclischen Restes oder

$R_1$ den Rest

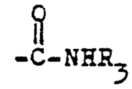

mit $R_3$ in der Bedeutung eines Alkanoyl- oder Alkansulfonylrestes mit je 1-10 C-Atomen oder des Restes $R_2$ oder

$R_1$ den Rest

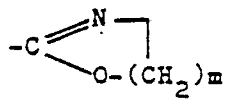

worin m die Zahl I oder 2 bedeutet,

X ein Sauerstoffatom oder eine $CH_2$-Gruppe,

A eine trans —CH = CH- oder —C ≡ C-Gruppe,

W eine freie oder funktionell abgewandelte Hydroxymethylengruppe, wobei die OH-Gruppe α- oder β-ständig sein kann,

n die Zahl 1, 2 oder 3 bedeuten kann,

D eine geradkettige Alkylengruppe mit 1-3-C-Atomen,

E eine —C ≡ C-Bindung oder eine —$CR_6$ = $CR_7$-Gruppe darstellt, wobei $R_6$ und $R_7$ sich unterscheiden und ein Wasserstoffatom oder eine Alkylgruppe mit 1-5 C-Atomen bedeuten oder ein Wasserstoffatom oder ein Halogenatom, vorzugsweise Chlor bedeuten,

$R_4$ eine Alkyl-, Cycloalkyl- oder eine gegebenenfalls substituierte Arylgruppe oder eine heterocyclische Gruppe,

$R_5$ eine freie oder funktionell abgewandelte Hydroxygruppe, und, falls $R_2$ die Bedeutung eines Wasserstoffatoms hat, deren Salze mit physiologisch verträglichen Basen bedeuten.

Die Verbindungen der Formel I stellen sowohl (5E)- als auch (5Z)-Isomere dar.

Als Alkylgruppen $R_2$ sind gerad- oder verzweigtkettige Alkylgruppen mit 1-10 C-Atomen zu betrachten, wie beispielsweise Methyl, Äthyl, Propyl, Isopropyl, Butyl, Isobutyl, tert.-Butyl, Pentyl, Isopentyl, Neopentyl, Heptyl, Hexyl, Decyl.

Die Alkylgruppen $R_2$ können gegebenenfalls 1 bis mehrfach substituiert sein durch Halogenatome, $C_1$-$C_4$-Alkoxygruppen, gegebenenfalls substituierte $C_6$-$C_{10}$-Arylgruppen, Di-$C_1$-$C_4$-alkylamine und Tri-$C_1$-$C_4$-alkylammonium. Bevorzugt sind solche Alkylgruppen, die einfach substituiert sind.

Als Substituenten seien beispielsweise genannt Fluor-, Chlor- oder Bromatome, Phenyl, Dimethylamino, Diäthylamino, Methoxy, Äthoxy. Als bevorzugte Alkylgruppen $R_2$ sind solche mit 1-7 C-Atomen, wie z. B. Methyl, Äthyl, Propyl, Dimethylaminopropyl, Isobutyl, Butyl zu nennen. Besonders bevorzugte Alkylgruppen $R_2$ sind solche mit 1-4 C-Atomen. Als Arylgruppen $R_2$ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, durch Chlormethyl-, Fluormethyl-, Trifluormethyl-, Carboxyl-, Hydroxy- oder Alkoxygruppen mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy. Die Cycloalkylgruppe $R_2$ kann im Ring 4-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl und Adamantyl. Als heterocyclische Gruppen $R_2$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl u. a. Der Aryl-Rest in der $-CH_2-\underset{\overset{\|}{O}}{C}-Aryl$ Gruppe von $R_2$ kann Phenyl, α- oder β-Naphthyl, die durch 1-3 Phenylgruppen, die wiederum durch 1-3 Halogenatome wie F, Cl oder Br oder 1-3 $C_1$-$C_4$-Alkoxygruppen oder 1-3 Halogenatome (F, Cl, Br) substituiert sein können, darstellen. Bevorzugt sind einfache Substitutionen mit Phenyl, $C_1$-$C_2$-Alkoxy, Chlor oder Brom.

Als Säurerest $R_3$ kommen physiologisch verträgliche Säurereste in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien $C_1$-$C_4$-Alkyl-, Hydroxy-, $C_1$-$C_4$-Alkoxy-, Oxo- oder Aminogruppen oder Halogenatome (F, Cl, Br) erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielsweise Methansulfonsäure, Äthansulfonsäure, Isopropansulfonsäure, β-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis-(β-chloräthyl)-aminosulfonsäure, N,N-Diisobutylaminosulfonsäure, N,N-Dibutylaminosulfonsäure, Pyrolidino-, Piperidino-, Piperazino-, N-Methylpiperazino- und Morpholinosulfonsäure in Frage.

Die Hydroxygruppen $R_5$ und in W können funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind.

Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.-butyl-silyl- und Tri-p-benzyl-silylrest. Als Acylreste kommen die gleichen wie für $R_3$ genannt in Frage; namentlich genannt seien beispielsweise Acetyl, Propionyl, Butyryl, Benzoyl.

Als Alkylgruppe $R_4$ kommen gerad- und verzweigtkettige, gesättigte und ungesättigte Alkylreste, vorzugsweise gesättigte, mit 1-10, insbesondere 1-7 C-Atomen, in Frage, die gegebenenfalls durch gegebenenfalls substituiertes Aryl substituiert sein können. Beispielsweise genannt seien Methyl-, Äthyl-, Propyl-, Butyl-, Isobutyl-, tert.-Butyl-, Pentyl-, Hexyl-, Heptyl-, Octyl-, Butenyl-, Isobutenyl-, Propenyl-, Pentenyl-, Hexenyl-, Benzyl- und p-Chlorbenzyl.

Die Cycloalkylgruppe $R_4$ kann im Ring 4-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methyl-cyclohexyl und Adamantyl.

Als substituierte bzw. unsubstituierte Arylgruppen $R_4$ kommen beispielsweise in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, $C_1$-$C_4$-Alkoxy- oder Hydroxygruppe. Bevorzugt ist die Substitution in 3- und 4-Stellung am Phenylring zum Beispiel durch Fluor, Chlor, $C_1$-$C_4$-Alkoxy oder Trifluormethyl oder in 4-Stellung durch Hydroxy. Als heterocyclische Gruppen $R_4$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl-, 4-Pyridyl, 3-Furyl, 3-Thienyl u. a.

Als Alkylengruppe D kommen geradkettige Alkylenreste, mit 1-5 C-Atomen, in Frage, wie z. B. Methylen, Äthylen, Propylen, Tetra- oder Pentamethylen.

Zur Salzbindung mit den freien Säuren ($R_2$ = H) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methyglucamin, Morpholin, Tris-(hydroxymethyl)-methylamin usw.

Die Erfindung betrifft ferner ein Verfahren zur Herstellung von Carbacyclinderivaten der allgemeinen Formel I, dadurch gekennzeichnet, daß man a) eine Verbindung der allgemeinen Formel II,

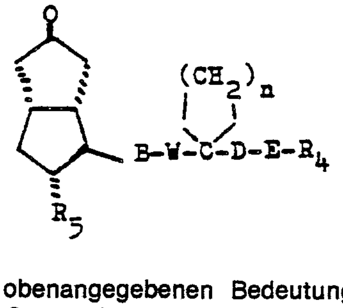

(II)

worin $R_5$, W, n, D, E und $R_4$ die obenangegebenen Bedeutungen aufweisen und B eine trans-Doppelbindung oder eine —CH = CBr-Gruppe bedeutet, gegebenenfalls nach Schutz anwesender freier Hydroxygruppen mit einem Wittig-Reagenz der Formel III

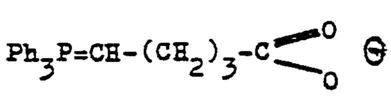

(III)

umsetzt oder b) eine Verbindung der allgemeinen Formel IV

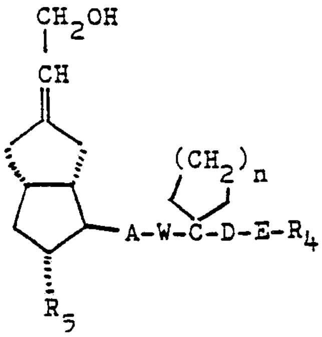

(IV)

worin $R_4$, $R_5$, A, W, D, E, n, die obenangegebenen Bedeutungen aufweisen, gegebenenfalls nach Schutz anwesender freier Hydroxygruppen mit einem Halogenessigsäurederivat der allgemeinen Formel V,

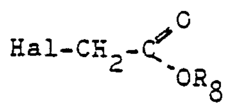

(V)

wobei Hal ein Chlor- Brom- oder Jodatom und $R_8$ einen Alkylrest oder Trialkyl-silylrest mit $C_1$-$C_4$-Alkylgruppen oder ein Alkalimetall (Na, Li, K) bedeutet, in Gegenwart einer Base veräthert und in den nach a) oder b) erhaltenen Verfahrensprodukten gegebenenfalls anschließend in beliebiger Reihenfolge Isomere trennt und/oder geschützte Hydroxygruppen freisetzt und/oder freie Hydroxygruppen verestert oder veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

Die Umsetzung der Verbindung der allgemeinen Formel II mit dem Wittig-Reagenz der Formel III, das man aus dem entsprechenden Phosphoniumsalz mit Methansulfinylmethylnatrium oder Methansulfinylkalium oder Kalium-ter.-butylat in Dimethylsulfoxid oder Dimethylsulfoxid-Tetrahydrofurangemischen herstellt, wird bei Temperaturen von 0 °C bis 100 °C, vorzugsweise 20 °C bis 60 °C, in einem aprotischen Lösungsmittel oder Lösungsmittelgemisch, vorzugsweise Dimethylsulfoxid, Dimethylformamid oder Tetrahydrofuran, vorgenommen. Die Trennung der dabei erhaltenen Z- und E-konfigurierten Olefine (5,6-Position) erfolgt auf übliche Art, beispielsweise durch Säulen- oder Schichtchromatographie. Bei der vorstehend beschriebenen Wittig-Olefinierung erfolgt, wenn B eine —CH = CBr-Gruppe bedeutet, gleichzeitig unter Abspaltung von Bromwasserstoff die Bildung der 13,14-Acetylenbindung.

Die Umsetzung der Verbindung der allgemeinen Formel IV mit einem Halogenessigsäurederivat der allgemeinen Formel V wird bei Temperaturen von 0 °C bis 100 °C, vorzugsweise 10 °C bis 80 °C, in einem aprotischen Lösungsmittel oder Lösungsmittelgemisch, beispielsweise Dimethylsulfoxid, Dimethylformamid, Tetrahydrofuran usw., vorgenommen. Als Basen kommen die dem Fachmann für Verätherungen bekannten Basen in Frage, beispielsweise Natriumhydrid, Kalium- tert.-butylat, Butyllithium usw.

Die Verseifung der Carbacyclinester wird nach den dem Fachmann bekannten Methoden durchgeführt, wie beispielsweise mit basischen Katalysatoren.

Die Einführung der Estergruppe $COOR_2$ für $R_1$, bei welcher $R_2$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid vermischt Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. *8*, Seiten 389-384 (1954)].

Die Einführung der Estergruppe $COOR_2$ für $R_1$, bei welcher $R_2$ eine substituierte oder unsubstituierte Arylgruppe darstellt, erfolgt nach den dem Fachmann bekannten Methoden. Beispielsweise werden die Carboxyverbindungen und die entsprechenden Arylhydroxyverbindungen mit Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Essigester, Tetrahydrofuran, vorzugsweise Chloroform, in Frage. Die Reaktion wird bei Temperaturen zwischen − 30 °C und + 50 °C, vorzugsweise bei + 10 °C, durchgeführt.

Die Einführung der Estergruppe $—COOR_2$ für $R_1$ kann auch durch Umsetzung des Carboxylatanions mit dem entsprechenden Alkylhalogenid oder Halogenketon ($Hal—CH_2—\overset{O}{\overset{\|}{C}}—AR$, wobei Ar Phenyl,

Diphenyl, die durch $C_1$-$C_2$-Alkoxy oder Chlor oder Brom substituiert sein können) erfolgen.

Die Einführung der $\Delta^2$-Oxazolingruppe

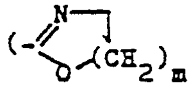

für $R_1$) erfolgt nach den in den DE-OS-3 047 759, 3 115 997 und 3 145 830 beschriebenen Verfahren. Beispielsweise werden die Carboxyverbindungen in Gegenwart des entsprechenden Aminoalkohols mit tert. Phosphinen, insbesondere Triphenylphosphin in Gegenart von Halogenverbindungen wie insbesondere Tetrachlorkohlenstoff und einer tert. Base, vorzugsweise Triäthylamin oder DBN, in die $\Delta^2$-Oxazoline überführt.

Die Carbacyclin-Derivate der allgemeinen Formel I mit $R_1$ in der Bedeutung einer Carboxygruppe ($R_2 = H$) können mit geeigneten Mengen der entsprechenden anorganischen Basen unter Neutralisierung in Salze überführt werden. Beispielsweise erhält man beim Lösen der entsprechenden Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die Carbacyclin-Säure zum Beispiel in einem geeigneten Lösungsmittel, wie Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0 °C-30 °C nach 15-30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u. a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u. a. oder in einer wäßrigen Lösung einer organischen Säure, wie zum Beispiel Salzsäure, durchgeführt. Zur Verbesserung der Löslichkeit wird zweckmäßigerweise ein mit Wasser misch bares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei − 10 °C bis 70 °C, vorzugsweise bei 25 °C.

Die Einführung der Amidgruppe $-\overset{O}{\overset{\|}{C}}NHR_3$ für $R_1$ erfolgt nach den dem Fachmann bekannten Methoden. Die Carbonsäuren der allgemeinen Formel I ($R_2 = H$) werden zunächst in Gegenwart eines tertiären Amins, wie beispielsweise Triäthylamin mit Chlorameisensäureisobutylester in das gemischte Anhydrid überführt. Die Umsetzung des gemischten Anhydrids mit dem Alkalisalz des entsprechenden Amids oder mit Ammoniak ($R_3 = H$) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen − 30 °C und + 60 °C, vorzugsweise bei 0 °C bis 30 °C.

Eine weitere Möglichkeit für die Einführung der Amidgruppe $-CONHR_3$ für $R_1$ besteht in der Umsetzung einer 1-Carbonsäure der allgemeinen Formel I ($R_2 = H$), in der freie Hydroxygruppen gegebenenfalls intermediär geschützt sind, mit Verbindungen der allgemeinen Formel VI,

$$O = C = N - R_3 \qquad \text{(VI)}$$

worin $R_3$ die obenangegebene Bedeutung hat.

Die Umsetzung der Verbindung der allgemeinen Formel I ($R_1$ = COOH) mit einem Isocyanat der allgemeinen Formel VI erfolgt gegebenenfalls unter Zusatz eines tertiären Amins, wie z. B. Triäthylamin oder Pyridin. Die Umsetzung kann ohne Lösungsmitel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen − 80 °C bis 100 °C, vorzugsweise bei 0 °C bis 30 °C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmässigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formeln II und IV können beispielsweise hergestellt werden, indem man in an sich bekannter Weise einen Aldehyd der Formel VII (DE-OS-2 845 770)

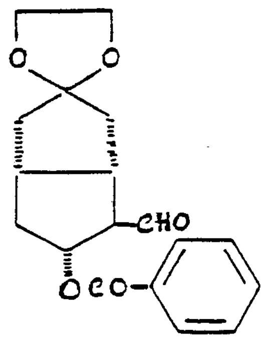

(VII)

mit einem Phosphonat der allgemeinen Formel VIII

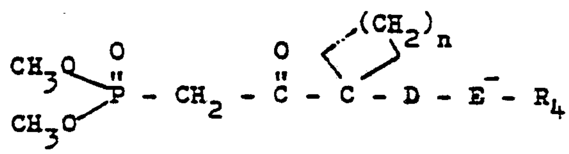

(VIII)

worin D, E und $R_4$ die obenangegebene Bedeutung aufweisen, in Gegenwart eines Deprotonierungsmittels, wie beispielsweise Natriumhydrid oder Kalium-tert.-butylat zu einem Keton der allgemeinen Formel IX (X = H) oder zusätzlich in Gegenwart eines Bromierungsmittels, wie beispielsweise N-Bromsuccinimid, zu einem Keton der allgemeinen Formel IX (X = Br) umsetzt.

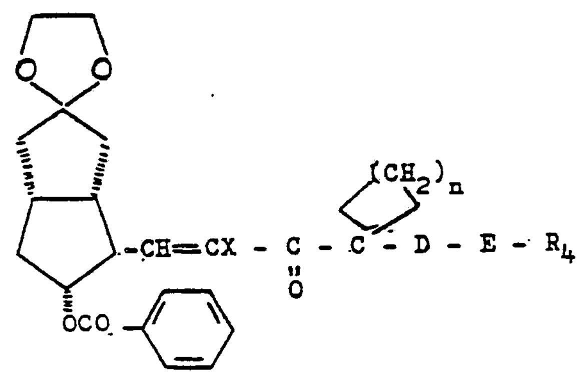

(IX)

Nach Reduktion der Ketogruppe mit Zinkborhydrid oder Natriumborhydrid oder Umsetzung mit Alkylmagnesiumbromid oder Alkyllithium and anschließender Epimerentrennung gelangt man zu den Verbindungen der allgemeinen Formel X

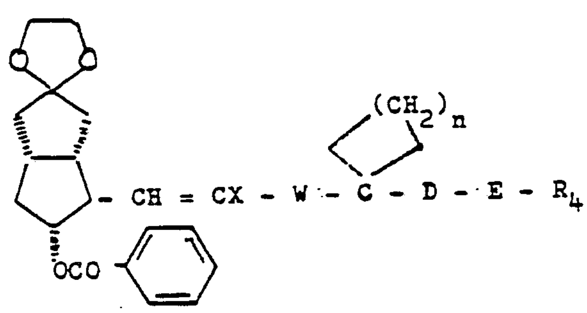

(X)

Verseifung der Estergruppe beispielsweise mit Kaliumcarbonat in Methanol sowie gegebenenfalls Hydrierung der Doppelbindung oder gegebenenfalls Verätherung mit Dihydropyran und anschließende Abspaltung von Bromwasserstoff mit beispielsweise Kalium-tert.-butylat in Dimethylsulfoxid, Ketalspaltung mit wässriger Essigsäure sowie gegebenenfalls funktionelle Abwandlung der freien Hydroxygruppen, beispielsweise durch Verätherung mit Dihydropyran liefert das Keton der allgemeinen Formel XI

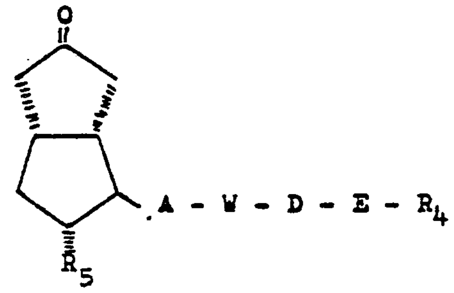

(XI)

Nach Olefinierungsreaktion mit Phosphonoessigsäuretriäthylester oder Phosphonoessigsäuretrimethylester und anschließender Reduktion mit Lithiumaluminiumhydrid erhält man die in der Doppelbindung isomeren Verbindungen der allgemeinen Formel IV die gegebenenfalls getrennt werden können.

Nach Reduktion der Ketogruppe mit Natriumborhydrid und gegebenenfalls Epimerentrennung, Verseifung der Estergruppe beispielsweise mit Kaliumcarbonat in Methanol und Ketalspaltung mit wäßriger Essigsäure sowie gegebenenfalls Epimerentrennung gelangt man zum Keton der allgemeinen Formel XII

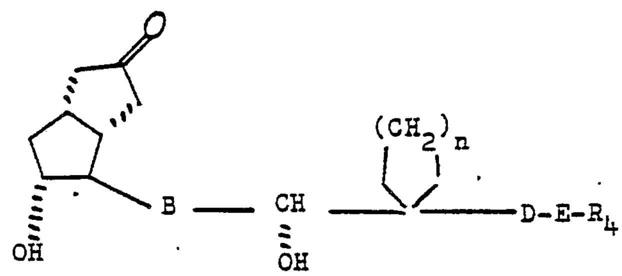

(XII)

Die Verätherung der Hydroxylgruppen mit z. B. Dihydropyran in Gegenwart katalytischer Mengen p-Toluolsulfonsäure liefert die Verbindungen der allgemeinen Formel II.

Die Herstellung der Phosphonate der allgemeinen Formel VIII erfolgt in an sich bekannter Weise durch Umsetzung eines Esters der allgemeinen Formel XIII

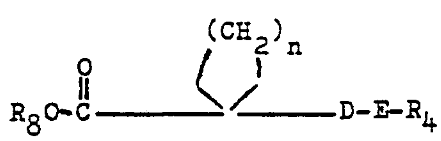

(XIII)

worin n, D, E, und $R_4$ die oben angegebene Bedeutung haben und $R_8$ eine Alkylgruppe mit 1-5 C-Atomen bedeutet, mit dem Anion von Methylphosphonsäuredimethylester.

Die Herstellung der Ester der allgemeinen Formel XIII folgt in an sich bekannter Weise durch Umsetzung eines Dianons XIV mit einer Halogenverbindung XV, wobei n, D, E, $R_4$ die obenangegebene Bedeutung haben und Hal ein Chlor-, Brom- oder Jodatom bedeutet.

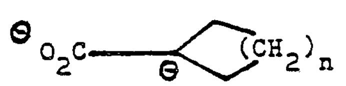

(XIV)

Hal - D - E - $R_4$

(XV)

Die Verbindungen dieser Erfindung wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoff dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere

Stabilität aus. Die hohe Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes, ohne zugleich Schlagvolumen und koronare Durchblutung zu senken ; Behandlung von Schlaganfall, Prophylaxe und Therapie koronarer Herzerkrankungen, koronarer Thrombose, des Herzinfarktes, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber und im Pankreas, antiallergische Eigenschaften, Senkung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclinderivate antiproliferative und antidiarrhoegene Eigenschaften. Die Carbacycline dieser Erfindung können auch in Kombination, z. B. mit β-Blockern oder Diuretika, verwendet werden.

Die Dosis der Verbindungen ist 1-1 500 µg/kg/Tag, wenn sie am menschlichen Patienten verabreicht werden. Die Einheitsdosis für den pharmazeutischen akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und herblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

## Beispiel 1

(5E) — 18,18,19,19-Tetradehydro-16,16-trimethylen-6a-carba-prostaglandin-$I_2$

Zu einer Lösung von 9,36 g 4-Carboxybutyltriphenylphosphoniumbromid in 20 ml Dimethylsulfoxid und 8 ml Tetrahydrofuran gibt man bei 5 °C innerhalb von 45 Minuten 4,74 g Kalium-tert.-butylat und rührt noch 45 Minuten bei 5 °C nach. Zur roten Ylenlösung fügt man eine Lösung von 1,65 g (1R, 5S, 6R, 7R)-7-(Tetrahydropyran-2-yloxy)-6-(E) (3R)-3-(tetrahydropyran-2-yloxy)-4,4-trimethylen-oct-1-en-6-inyl]-bicyclo [3.3.0] octan-3-on in 2 ml Tetrahydrofuran und rührt 3 Stunden bei 35 °C. Das Reaktionsgemisch wird auf Eiswasser gegossen, mit Zitronensäure auf $pH_4$ angesäuert und mit Methylenchlorid extrahiert. Die organische Phase wird mit Sole geschüttelt, über Magnesiumsulfat getrocknet und im Vakuum eingedampft. Den Rückstand reinigt man durch Chromatographie. Mit Hexan/Essigester (1 : 1) erhält man zunächst 280 mg des Z-konfigurierten Olefins sowie als polarere Komponente 700 mg [(5E)-18,18,19,19-Tetradehydro-16,16-trimethylen-6a-carba-prostaglandin-$I_2$-11,15-bis-(tetrahydropyranyläther) als farbloses Öl

IR : ($CHCl_3$) : 3 520, 2 940, 2 860, 1 710, 1 073, 1 019, 972/cm

Zur Schutzgruppenspaltung rührt man 650 mg des vorstehend erhaltenen Produktes mit 20 ml einer Mischung aus Essigsäure/Wasser/Tetrahydrofuran (65/35/10) 24 Stunden bei 25 °C, dampft im Vakuum ein und chromatographiert den Rückstand an Kieselgel. Mit Essigester/0,1 %Essigsäure erhält man 320 mg der Titelverbindung als farbloses Öl.

IR : 3 600, 3 520, 3 410 (breit), 2 937, 2 860, 1 710, 972/cm

1 a) 2-Oxo-3,3-trimethylen-hept-5-in-phosphonsäure-dimethylester

Zu einer Lösung von 44,52 g Diisopropylamin in 200 ml Tetrahydrofuran gibt man bei − 30 °C 265 ml 1,66 M Butyllithium-Lösung in Hexan und anschließend 20,02 g Cyclobutancarbonsäure. Man rührt noch 30 Minuten bei − 10 °C und tropft dann 31,92 g 1-Brom-2-butin zu, rührt 16 Stunden bei 25 °C und gießt auf 600 ml Eiswasser. Nach Ansäuern mit 2 N Salzsäure auf pH 4 wird mit Äther extrahiert, der Extrakt mit Sole gewaschen, über Magnesiumsulfat getrocknet und im Vakuum der Äther abgedampft. Der

Rückstand wird durch Destillation gereinigt. Man erhält 18 g 2,2-Trimethylen-hex-4-in-Säure (Kp 140°-143°, 15 Torr).

IR : 3 520, 2 985, 2 961, 1 700, 1 425, 1 408, 1 100, 1 035, 920, 880/cm

Zur Veresterung kocht man 14 g der so erhaltenen Carbonsäure in 200 ml Methanol und 2 ml conc. Schwefelsäure 6 Stunden unter Rückfluß. Man kühlt ab, gab auf 150 g Eis und extrahiert mit Äther. Der Extrakt wird mit Sodalösung und Sole gewaschen, über Magnesiumsulfat getrocknet und der Äther im Vakuum abgedampft. Man erhält nach Destillation (Kp 92-94° bei 15 Torr) 13 g 2,2-Trimethylen-hex-4-in-Säuremethylester.

IR : 2 985, 2 951, 2 92L, 2 858, 1 725, 1 431, 1 330, 1 099, 978, 875/cm

Zu einer Lösung von 21,35 g Methanphosphonsäuredimethylester in 300 ml Tetrahydrofuran tropft man bei − 70 °C 200 ml 1,7 M Butyllithiumlösung in Hexan. Nach 20 Minuten wird eine Lösung von 13 g des vorstehend hergestellten Esters in 50 ml Tetrahydrofuran zugetropft und weiter 5 Stunden bei − 70° gerührt. Anschließend wird mit 12 ml Essigsäure versetzt und im Vakuum weitgehend eingedampft. Der Rückstand wird mit 50 ml Wasser gelöst und mehrmals mit Dichlormethan extrahiert. Man trocknet über Magnesiumsulfat und dampft im Vakuum ein. Nach Destillation des Rückstandes (Kp 133°-139 °C bei 0,5 Torr) erhält man 15,1 g der Titelverbindung als farblose Flüssigkeit.

IR : 3 420 (breit), 2 988, 2 952, 2 850, 1 702, 1 251, 1 030/cm

1 b) (1R,5S,6R,7R)-3,3-Äthylendioxy-7-benzoyloxy-6-[(E)-3-oxo-4,4-trimethylen-oct-1-en-6-inyl]-bicyclo-[3.3.0] octan

Zu einer Suspension von 980 mg Natriumhydrid in 200 ml Dimethoxyäthan tropft man bei 0 °C eine Lösung von 11,62 g des nach Beispiel 1 a hergestellten Phosphonats in 120 ml Dimethoxyäthan und rührt 1 Stunde bei 0 °C. Anschließend tropft man eine Lösung von 11,40 g (1R,5S,6R,7R)-3,3-Äthylendioxy-7-benzoyloxy-6-formylbicyclo [3.3.0] octan in 120 ml Dimethoxyäthan ein. Nach 2 Stunden bei 0 °C gießt man auf 800 ml gesättigte Ammoniumchloridlösung und extrahiert mit Äther, wäscht den Extrakt mit Sole, trocknet über Magnesiumsulfat, und dampft im Vakuum ein. Nach Chromatographie des Rückstandes an Kieselgel mit Hexan/Äther (1 : 1) erhält man 9,10 g der Titelverbindung als farbloses Öl.

IR : 2 940, 2 859, 1 712, 1 686, 1 621, 1 600, 1 584, 1 270, 980, 942/cm

1 c) (1R,5S,6R,7R)-3,3-Äthylendioxy-7-benzoyloxy-6-[(E) (3R)-3-hydroxy-4,4-trimethylen-oct-1-en-6-inyl]-bicyclo [3.3.0] octan

Eine Lösung von 8,10 g des nach Beispiel 1 b hergestellten Ketons in 200 Methanol wird bei − 40 °C mit 3,60 g Natriumborhydrid versetzt. Anschließend rührt man 30 Minuten bei − 40 °C, verdünnt dann mit 2 l Äther, schüttelt mehrmals mit je 50 ml Sole, trocknet, über Magnesiumsulfat und dampft im Vakuum ein. Der Rückstand wird durch Chromatographie an Kieselgel mit Äther/Hexan (7 + 3) gereinigt. Man erhält 4,9 g der Titelverbindung als farbloses Öl.

IR : 3 600, 3 520, 2 938, 2 881, 1 713, 1 601, 1 586, 1 275, 970, 943/cm

Außerdem erhält man 3,55 g der 15β-Hydroxy-Verbindung als polare Komponente (PG-Nummerierung).

1 d) (1R,5S,6R,7R)-7-(Tetrahydropyran-2-yloxy)-6-[(E) (3R)-3-(tetrahydropyran-2-yloxy)-4,4-trimethylenoct-1-en-6-inyl]-bicyclo [3.3.0] octan-3-on

Eine Lösung von 4,90 g der nach Beispiel 1 c hergestellten Verbindung in 150 ml Methanol rührt man über Nacht mit 2,25 g wasserfreiem Kaliumcarbonat. Man engt im Vakuum ein, nimmt in 1 l Äther auf, schüttelt mit Wasser und trocknet über $MgSO_4$. Nach Abdampfen des Äthers erhält man 4,75 g rohes Diol, das man 20 Stunden mit 170 ml einer Mischung aus Essigsäure/Wasser/Tetrahydrofuran (65/35/10) bei Raumtemperatur rührt. Anschließend dampft man im Vakuum ein und reinigt den Rückstand durch Chromatographie an Kieselgel mit Hexan/Essigester (3 + 7). Dabei erhält man 2,95 g des Keto-diols, das man in 100 ml Dichlormethan löst und bei 0 °C mit 4 g Dihydropyran und 30 mg p-Toluolsulfonsäure versetzt. Man rührt 30 Minuten bei 0 °C, gießt auf 50 ml Natriumhydrogencarbonatlösung, extrahiert mit Dichlormethan, trocknet über Magnesiumsulfat und dampft im Vakuum ein. Den Rückstand chromatographiert man an Kieselgel mit Hexan/Essigester (7 + 3) und erhält 3,92 g der Titelverbindung als farbloses Öl.

IR : 2 941, 2 868, 1 738, 972/cm

## Beispiel 2

(5E)-13,14-Didehydro-18,18,19,19-tetradehydro-16,16-trimethylen-6a-carba-prostaglandin-$I_2$

Zu einer Lösung von 9,40 g 4-Carboxybutyltriphenylphosphoniumbromid in 20 ml Dimethylsulfoxid und 8 ml Tetrahydrofuran fügt man bei 5 °C innerhalb von 45 Minuten 4,75 g Kalium-tert-butylat und rührt 45 Minuten bei 5 °C nach. Zur roten Ylenlösung fügt man eine Lösung von 1,95 g (1R, 5S, 6R, 7R)-7-(tetrahydropyran-2-yloxy)-6-[(3S)-2-brom-3-(tetrahydropyran-2-yloxy)-4,4-trimethylen-oct-1-en-6-inyl]-bicyclo [3.3.0] octan-3-on in 4 ml Tetrahydrofuran und rührt 4 Stunden bei 40 °C. Man gießt dann auf Eiswasser, säuert mit Zitronensäure auf pH 4 an, extrahiert mit Dichlormethan, wäscht den Extrakt mit Sole, trocknet über Magnesiumsulfat und dampft in Vakuum ein. Den Rückstand reinigt man durch Chromatographie an Kieselgel. Mit Hexan/Essigester (3 + 2) erhält man zunächat 200 mg des 5Z-konfigurierten Olefins, sowie als polarere Komponente 730 mg (5E)-13,14-Didehydro-18,18,19,19-tetradehydro-16,16-trimethylen-6a-carba-prostaglandin-$I_2$-11,15-bis-(tetrahydropyranyläther) als farbloses Öl.

IR : 3 520, 2 941, 2 860, 1 710, 1 020/cm

Zur Abspaltung der Schutzgruppen rührt man 700 mg des vorstehend erhaltenen Produktes mit 25 ml einer Mischung aus Essigsäure/Wasser/Tetrahydrofuran (65/35/10) über Nacht bei 25 °C, dampft im Vakuum ein und chromatographiert den Rückstand an Kieselgel mit Essigester/0,1 % Essigsäure. Man erhält 350 mg der Titelverbindung als farbloses Öl.

IR : 3 600, 3 405 (breit), 2 930, 2 221, 1 711, 1 603, 1 012/cm

Das Ausgangsmaterial für die obige Titelverbindung wird wie folgt hergestellt :

2a) (1R,5S,6R,7R)-3,3-Äthylendioxy-7-benzoyloxy-6-(2-brom-3-oxo-4,4-trimethylen-oct-1-en-6-inyl)-bicyclo-[3.3.0] octan

Zu einer Suspension von 1,81 g Natriumhydrid (55 %-ig in Mineralöl) in 180 ml Dimethoxyäthan tropft man bei 0 °C eine Lösung von 11,8 g 2-Oxo-3,3-trimethylenhept-5-in-phosphonsäuredimethylester in 75 ml Dimethoxyäthan, rührt eine Stunde bei 0 °C und fügt dann 7,40 g fein gepulvertes N-Bromsuccinimid hinzu. Man rührt 30 Minuten bei 0 °C, versetzt mit einer Lösung aus 11,40 g (1R,5S,6R,7R)-3,3-Äthylendioxy-7-benzoyloxy-6-formyl-bicyclo [3.3.0] octan in 90 ml Dimethoxyäthan und rührt 2 Stunden bei 0 °C. Anschließend gießt man auf gesättigte Ammoniumchloridlösung und extrahiert mit Äther. Man wäscht den Extrakt mit Sole neutral, trocknet über Magnesiumsulfat und dampft in Vakuum ein. Nach Chromatographie des Rückstandes an Kieselgel mit Hexan/Äther (1 + 1) erhält man 8,92 g der Titelverbindung als Öl.

IR : 2 935, 2 878, 1 712, 1 690, 1 602, 1 588, 1 450, 1 275, 944/cm

2b) (1R,5S,6R,7R)-7-Hydroxy-6-[(3S)-2-brom-3-hydroxy-4,4-trimethylen-oct-l-en-6-inyl]-bicyclo [3.3.0] octan-3-on

Zu einer Lösung von 8,50 g des nach Beispiel 2 a hergestellten Ketons in 175 ml Methanol fügt man bei − 40 °C portionsweise 3,60 g Natriumborhydrid und rührt 30 Minuten bei − 40 °C. Anschließend verdünnt man mit Äther, wäscht mit Sole neutral, trocknet über Magnesiumsulfat und dampft in Vakuum ein. Das Rohprodukt (Epimerengemisch) löst man in 250 ml Methanol, fügt 3,60 g Kaliumcarbonat zu und rührt über Nacht bei 25 °C. Anschließend engt man im Vakuum ein, verdünnt mit Äther und wäscht mit Sole neutral. Man trocknet über Magnesiumsulfat und dampft im Vakuum ein. Den Rückstand rührt man 16 Stunden bei 25 °C mit 300 ml einer Mischung aus Essigsäure/Wasser/Tetrahydrofuran (65/35/10) und dampft anschließend im Vakuum ein. Durch Chromatographie an Kieselgel mit Äther/Dichlormethan erhält man 2,10 g der 15β-Hydroxy-Verbindung (PG-Nummerierung) sowie als polarere Komponente 2,90 g der Titelverbindung (15α-Hydroxy) als farbloses Öl.

IR : 3 600, 3 420 (breit), 2 957, 2 931, 1 738, 1 600, 1 405/cm

2c) (1R,5S,6R,7R)-7-(tetrahydropyran-2-yloxy)-6-[(3S)-2-brom-3-(tetrahydropyran-2-yloxy)-4,4-trimethylenoct-l-en-6-inyl]-bicyclo [3.3.0] octan -3-on

Eine Lösung von 2 g des nach Beispiel 2 b hergestellten α-Alkohols, 20 mg p-Toluolsulfonsäure und 2 g Dihydropyran in 50 ml Dichlormethan rührt man 30 Minuten bei 0 °C. Anschließend verdünnt man mit Dichlormethan, schüttelt mit Natriumhydrogencarbonatlösung und Sole, trocknet über Magnesiumsulfat und dampft im Vakuum ein. Nach Chromatographie des Rückstandes an Kieselgel erhält man mit Hexan/Äther(6 + 4) 2,60 g der Titelverbindung als farbloses Öl.

IR : 2 938, 2 865, 1 735, 1 452, 1 121/cm

## Beispiel 3

(5E)-20-Methyl-18,18,19,19-tetradehydro-16,16-trimethylen-6a-carba-prostaglandin-$I_2$

In Analogie zu Beispiel 1 erhält man aus 1,50 g (1R,5S,6R,7R)-7-(Tetrahydropyran-2-yloxy)-6-[(E)-(3R)-3-(tetrahydropyran-2-yloxy)-4,4-trimethylen-non-1-en-6-inyl]-bicyclo [3.3.0] octan-3-on 630 mg (5E)-20-Methyl-18,18,19,19-tetrahydro-16,16-trimethylen-6a-carbaprostaglandin-$I_2$-11,15-bis-(tetrahydropyranyläther) als farbloses Öl.

IR : 3 520, 2 940, 2 862, 1 712, 1 070, 1 020, 974/cm

Nach Abspaltung der Schutzgruppen gemäß Beispiel 1 erhält man 310 mg der Titelverbindung als farbloses Öl.

IR : 3 600, 3 520, 3 405 (breit), 2 938, 2 862, 1 710, 972/cm

Die Ausgangsmaterialien für die obige Titelverbindung werden wie folgt hergestellt :

3 a) 2-Oxo-3,3-trimethylen-oct-5-in-phosphonsäure-dimethylester

In Analogie zu Beispiel I erhält man aus Cyclobutancarbonsäure und 1-Brom-2-pentin die 2,2-Trimethylen-hept-4-in-Säure ($Kp_{15}$ 159-160 °C), die nach Veresterung den Methylester liefert ($Kp_{15}$ 111-112 °C). Umsetzung mit der Lithiumverbindung von Methanphosphonsäuredimethylester gemäß Beispiel 1 a führt zur Titelverbindung, Kp 145-147 °C bei 0,3 Torr.

IR : 3 420 (breit), 2 982, 2 948, 2 851, 1 702, 1 250, 1 028/cm

3 b) (1R,5S,6R,7R)-3,3-Äthylendioxy-7-benzoyloxy-6-[(E)-3-oxo-4,4-trimethylen-non-1-en-6-inyl] bicyclo [3.3.0] octan

In Analogie zu Beispiel 1 b erhält man aus 11,40 g (1R,5S,6R,7R)-3,3-Äthylendioxy-7-benzoyloxy-6-formyl-bicyclo [3.3.0] octan mit 2-Oxo-3,3-trimethylen-oct-5-in-phosphonsäuredimethylester 10,02 g der Titelverbindung als farbloses Öl.

IR : 2 942, 2 861, 1 712, 1 688, 1 620, 1 600, 1 585, 1 270, 981, 944/cm

3 c) (1R,5S,6R,7R)-3,3-Äthylendioxy-7-benzoyloxy-6-[(E)-(3R)-3-hydroxy-4,4-trimethylen-non-1-en-6-inyl]- bicyclo [3.3.0] octan

In Analogie zu Beispiel 1 c erhält man aus 10 g der nach Beispiel 3 b hergestellten Verbindung 5,95 g der Titelverbindung als farbloses Öl.

IR : 3 600, 3 520, 2 937, 2 880, 1 712, 1 601, 1 585, 1 270, 972, 948/cm

3 d) (1R,5S,6R,7R)-7-(Tetrahydropyran-2-yloxy)-6-[(E)-(3R)-3-(tetrahydropyran-2-yloxy)-4,4-trimethylen-non-1-en-6-inyl]-bicyclo [3.3.0] octan-3-on

In Analogie zu Beispiel 1 d erhält man aus 5,50 g der nach Beispiel 3 c hergestellten Verbindung durch Esterspaltung, Ketalspaltung und Schutz der Hydroxygruppen als Bis-tetrahydropyranyläther 4,25 g der Titelverbindung als farbloses Öl.

IR : 2 940, 2 865, 1 738, 976/cm

## Beispiel 4

(5E)-20-Äthyl-18,18,19,19-tetradehydro-16,16-trimethylen-6a-carba-prostaglandin-$I_2$

In Analogie zu Beispiel 1 erhält man aus 700 mg (1R,5S,6R,7R)-7-Tetrahydropyran-2-yloxy-6-[(E)-(3R)-3-(tetrahydropyran-2-yloxy)-4,4-trimethylen-dec-1-en-6-inyl]-bicyclo [3.3.0] octan-3-on 270 mg (5E)-20-Äthyl-18,18,19,19-tetrahydro-16,16-trimethylen-6a-carba-prostaglandin-$I_2$-11,15-bis-tetrahydropyranyläther als farbloses Öl.

IR : 3 520, 2 945, 2 870, 1 711, 1 072, 1 020, 976/cm

Nach Abspaltung der Schutzgruppen gemäß Beispiel 1 erhält man 140 mg der Titelverbindung als farbloses Öl.

IR : 3 600, 3 505, 3 410 (breit), 2 940, 1 711, 976/cm

Die Ausgangsmaterialien für die obige Titelverbindung werden wie folgt hergestellt :

4 a) 2-Oxo-3,3-trimethylen-non-5-in-phosphonsäure-dimethylester

In Analogie zu Beispiel 1 a erhält man aus Cyclobutansäure und 1-Brom-2-hexin die 2,2-Trimethylenoct-4-in-Säure (Kp 178-180 °C bei 14 Torr), die nach Veresterung den Methylester liefert (Kp 128-130 °C bei 15 Torr). Umsetzung mit der Lithiumverbindung von Methanphosphonsäuredimethylester gemäß Beispiel 1 a führt zur Titelverbindung (Kp 150-152 °C bei 0,1 Torr).

IR : 3 420 (breit), 2 986, 2 951, 2 850, 1 704, 1 250, 1 031/cm

4 b) (1R,5S,6R,7R)-3,3-Äthylendioxy-7-benzoyloxy-6-[(E)-3-oxo-4,4-trimethylen-dec-1-en-6-inyl]-bicyclo [3.3.0] octan

In Analogie zu Beispiel 1 b erhält man aus 10,0 g (1R,5S,6R,7R)-3,3-Äthylendioxy-7-benzoyloxy-6-formyl-bicyclo [3.3.0] octan mit 2-Oxo-3,3-trimethylen-non-5-in-phosphonsäuredimethylester 8,50 g der Titelverbindung als farbloses Öl.

IR : 2 945, 2 860, 1 712, 1 687, 1 601, 1 585, 1 269, 978, 948/cm

4 c) (1R,5S,6R,7R)-3,3-Äthylendioxy-7-benzoyloxy-6-[(E)-(3R)-3-hydroxy-4,4-trimethylen-dec-1-en-6-inyl]-bicyclo [3.3.0] octan

In Analogie zu Beispiel 1 c erhält man aus 8,00 g der nach Beispiel 4 b hergestellten Verbindung 4,85 g der Titelverbindung als farbloses Öl.
IR : 3 600, 3 510, 2 940, 2 881, 1 712, 1 600, 1 585, 1 272, 974, 946/cm

4 d) (1R,5S,6R,7R)-7-(Tetrahydropyran-2-yloxy-6-[(E)-(3R)-3-(tetrahydropyran-2-yloxy)-4,4-trimethylendec-1-en-6-inyl]-bicyclo [3.3.0] octan-3-on

In Analogie zu Beispiel 1 d erhält man aus 4,00 g der nach Beispiel 4 c hergestellten Verbindung durch Esterspaltung, Ketalspaltung und Schutz der Hydroxygruppen als Bis-tetrahydropyranyläther 3,10 g der Titelverbindung als farbloses Öl.
IR : 2 940, 2 860, 1 738, 974/cm

Beispiel 5

(5E)-13,14-Didehydro-20-methyl-18,18,19,19-tetradehydro-16,16-trimethylen-6a-carba-prostaglandin-$I_2$

Zu einer Lösung von 4,71 g 4-Carboxybutyltriphenylphosphoniumbromid in 10 ml Dimethylsulfoxid und 5 ml Tetrahydrofuran fügt man bei 5 °C innerhalb von 45 Minuten 2,38 g Kalium-tert-butylat und rührt 45 Minuten bei 5 °C nach. Zur roten Ylenlösung tropft man eine Lösung von 1,00 g (1R, 5S, 6R, 7R)-7-(tetrahydropyran-2-yloxy)-6-[(3S)-2-brom-3-(tetrahydropyran-2-yloxy)-4,4-trimethylen-non-1-en-6-inyl]-bicyclo [3.3.0] octan-3-on in 2 ml Tetrahydrofuran und rührt 4 Stunden bei 40 °C. Man gießt auf Eiswasser, säuert mit Zitronensäure auf pH 4 an, extrahiert mit Dichlormethan, wäscht den Extrakt mit Sole, trocknet über Magnesiumsulfat und dampft im Vakuum ein. Den Rückstand reinigt man durch Chromatographie an Kieselgel. Mit Hexan/Essigester (1 : 1) erhält man zunächst 170 mg (5Z)-13,14-Didehydro-20-methyl-18,18,19,19-tetradehydro-16,16-trimethylen-6a-carba-prostaglandin-$I_2$-11,15-bis-tetrahydropyranyläther und als polarere Komponente 430 mg der entsprechenden 5-E-Verbindung, die man zur Abspaltung der Schutzgruppen mit 15 ml einer Mischung aus Essigsäure/Wasser/Tetrahydrofuran (65/35/10) 16 Stunden bei 25 °C rührt. Man dampft ein und reinigt den Rückstand durch Chromatographie an Kieselgel mit Essigester und erhält 190 g der Titelverbindung als farbloses Öl.
IR : 3 600, 3 410 (breit), 2 935, 1 712, 1 602, 1 015/cm
Das Ausgangsmaterial für die obige Titelverbindung wird wie folgt hergestellt :

5 a) (1R,5S,6R,7R)-3,3-Äthylendioxy-7-benzoyloxy-6-(2-brom-3-oxo-4,4-trimethylen-non-1-en-6-inyl)-bicyclo [3.3.0] octan

In Analogie zu Beispiel 2 a erhält man aus 6,49 g 2-Oxo-3,3-trimethylen-oct-5-in-phosphonsäuredimethylester, 3,70 g N-Bromsuccinimid und 5,70 g (1R,5S,6R,7R)-3,3-Äthylendioxy-7-benzoyloxy-6-formylbicyclo [3.3.0] octan 5,10 g der Titelverbindung als Öl.
IR : 2 942, 2 877, 1 712, 1 688, 1 600, 1 585, 1 451, 1 270, 948/cm

5 b) (1R,5S,6R,7R)-7-Hydroxy-6-[(3S)-2-brom-3-hydroxy-4,4-trimethylen-non-1-en-6-inyl]-bicyclo [3.3.0] octan-3-on

In Analogie zu Beispiel 2 b erhält man aus 5 g der nach Beispiel 5 a hergestellten Verbindung durch Reduktion mit Natriumborhydrid, Abspaltung des Benzoats mit Kaliumcarbonat in Methanol und Ketalspaltung 1,75 g der Titelverbindung als farbloses Öl.
IR : 3 600, 3 410 (breit), 2 955, 2 930, 1 738, 1 600, 1 407/cm

5 c) (1R,5S,6R,7R)-7-(tetrahydropyran-2-yloxy)-6-[(3S)-2-brom-3-(tetrahydropyran-2-yloxy)-4,4-trimethylen-non-1-en-6-inyl]-bicyclo [3.3.0] octan-3-on

In Analogie zu Beispiel 2 c erhält man aus 1,50 g des nach Beispiel 5 b hergestellten Diols mit Dihydropyran und p-Toluolsulfonsäure 1,95 g der Titelverbindung als farbloses Öl.
IR : 2 940, 2861, 1 738, 1 450, 1 120/cm

Beispiel 6

(5Z)-13,14-Didehydro-20-methyl-18,18,19,19-tetradehydro-16,16-trimethylen-6a-carba-prostaglandin-$I_2$

Man rührt 160 mg (5Z)-13,14-Didehydro-20-methyl-18,18,19,19-tetradehydro-16,16-trimethylen-6a-carba-prostaglandin-$I_2$

Man rührt 160 mg (5Z)-13,14-Didehydro-20-methyl, 18,18,19,19-tetradehydro-16,16-trimethylen-6a-carba-prostaglandin-$I_2$-11,15-bis-tetrahydropyranyläther (hergestellt nach Beispiel 5) mit 5 ml einer Mischung aus Essigsäure/Wasser/Tetrahydrofuran (65/35/10) 16 Stunden bei 25 °C. Anschließend dampft man im Vakuum ein und reinigt den Rückstand durch Chromatographie an Kieselgel mit Essigester und erhält 75 mg der Titelverbindung als farbloses Öl.

IR : 3 600, 3 410 (breit), 2 938, 2 221, 1 712, 1 600, 1 015/cm

Beispiel 7

(5E)-20-Äthyl-13,14-didehydro-18,18,19,19-tetradehydro-16,16-trimethylen-6a-carba-prostaglandin-$I_2$

In Analogie zu Beispiel 2 erhält man aus 1,50 g (1R,5S,6R,7R)-7-Tetrahydropyran-2-yloxy-6- [(3S)-2-brom-3-(tetrahydropyran-2-yloxy)-4,4-trimethylen-dec-1-en-6-inyl]-bicyclo [3.3.0] octan-3-on 270 mg der Titelverbindung als farbloses Öl.

IR : 3 600, 3 410 (breit), 2 932, 2 226, 1 712, 1 601, 1 015/cm

Das Ausgangsmaterial für die obige Titelverbindung wird wie folgt hergestellt :

7 a) (1R,5S,6R,7R)-3,3-Äthylendioxy-7-benzoyloxy-6-(2-brom-3-oxo-4,4-trimethylen-dec-1-en-6-inyl)-bicyclo [3.3.0] octan

In Analogie zu Beispiel 2 a erhält man aus 12,9 g 2-Oxo-3,3-trimethylen-non-5-in-phosphonsäuredimethylester, 7,40 gN-Bromsuccinimid und 11,40 g (1R,5S,6R,7R)-3,3-Äthylendioxy-7-benzoyloxy-6-formyl-bicyclo [3.3.0] octan 11,25 g der Titelverbindung als Öl.

IR : 2 941, 2 875, 1 711, 1 690, 1 602, 1 586, 1 451, 1 275, 948/cm

7 b) (1R,5S,6R,7R)-7-Hydroxy-6-[(3S)-2-brom-3-hydroxy-4,4-trimethylen-dec-1-en-6-inyl]-bicyclo [3.3.0] octan-3-on

In Analogie zu Beispiel 2 b erhält man aus 10 g des nach Beispiel 7 a hergestellten Ketons 2,52 g der Titelverbindung als farbloses Öl.

IR : 3 600, 3 420 (breit), 2 955, 2 930, 1 738, 1 600, 1 402/cm

7 c) (1R,5S,6R,7R)-7-(tetrahydropyran-2-yloxy/-6-[(3S)-2-brom-3-(tetrahydropyran-2-yloxy)-4,4-trimethylen-dec-1-en-6-inyl]-bicyclo [3.3.0] octan-3-on

In Analogie zu Beispiel 2 c erhält man aus 2 g des nach Beispiel 7 b hergestellten Diols 2,45 g der Titelverbindung als farbloses Öl.

IR : 2 942, 2 862, 1 737, 1 453, 1 120/cm

Beispiel 8

(5E)-20-Methyl-19,19,20,20-tetradehydro-16,16-trimethylen-6a-carba-prostaglandin-$I_2$

In Analogie zu Beispiel 1 erhält man aus 1,50 g (1R,5S,6R,7R)-7-(Tetrahydropyran-2-yloxy)-6 [(E)-(3S)-3-(tetrahydropyran-2-yloxy)-4,4-trimethylen-non-1-en-7-inyl]-bicyclo [3.3.0]-octan-3-on 300 mg der Titelverbindung als farbloses Öl.

IR : 3 600, 3 405 (breit), 2 938, 2 855, 1 711, 976/cm

8 a) 2-Oxo-3,3-trimethylen-oct-6-in-phosphonsäuredimethylester

Man verfährt zunächst analog Beispiel 1 a und erhält aus Cyclobutancarbonsäure und 1-Brom-3-Pentin die 2,2-Trimethylen-hept-5-in-Säure (Kp 155-158 °C bei 14 Torr) und durch Veresterung mit Methanol/Schwefelsäure den 2,2-Trimethylen-hept-5-in-Säuremethylester (Kp 108-110 °C bei 15 Torr).

IR : 2 965, 2 950, 2 921, 2 849, 1 728, 1 430, 1 330, 1 097, 970, 870/cm

und daraus mit Methanphosphonsäuredimethytelester gemäß Beispiel 1 a die Titelverbindung als farblose Flüssigkeit, Kp 148-150 °C bei 0,3 Torr.

IR : 3 420 (breit), 2 990, 2 954, 2 848, 1 702, 1 250, 1 030/cm

8 b) (1R,5S,6R,7R)-3,3-Äthylendioxy-7-benzoyloxy-6-[(E)-3-oxo-4,4-trimethylen-non-1-en-7-inyl] bicyclo [3.3.0] octan

In Analogie zu Beispiel 1 a erhält man aus 10 g (1R,5S,6R,7R)-3,3-Äthylendioxy-7-benzoyloxy-6-formyl-bicyclo [3.3.0] octan und dem nach Beispiel 8 a hergestellten Phosphonat 11,30 g der Titelverbindung als farbloses Öl.

IR : 2 942, 2 860, 1 710, 1 688, 1 620, 1 600, 1 585, 1 275, 980, 948/cm

8 c) (1R,5S,6R,7R)-3,3-Äthylendioxy-7-benzoyloxy-6-[(E)-(3R)-3-hydroxy-4,4-trimethylen-non-1-en-7-inyl]-bicyclo [3.3.0] octan

In Analogie zu Beispiel 1 c erhält man aus 11,0 g des nach Beispiel 8 b hergestellten Ketons 7,51 g der Titelverbindung (15α-Hydroxy) als farbloses Öl.
IR : 3 600, 3 510, 2 940, 2 881, 1 712, 1 600, 1 585, 1 275, 974, 947/cm

8 d) (1R,5S,6R,7R)-7-(Tetrahydropyran-2-yloxy)-6-[(E)-(3R)-(tetrahydropyran-2-yloxy)-4,4-trimethylen-non-1-en-7-inyl]-bicyclo [3.3.0] octan-3-on

In Analogie zu Beispiel 1 d erhält man aus 5 g des nach Beispiel 8 c hergestellten 15α-Alkohols 4,05 g der Titelverbindung als farbloses Öl.
IR : 2 940, 2 862, 1 738, 974/cm

## Beispiel 9

(5E)-13,14-Didehydro-20-methyl-19,19,20,20-tetrahydro-16,16-trimethylen-6a-carba-prostaglandin-$I_2$

In Analogie zu Beispiel 2 erhält man aus 500 mg (1R,5S,6R,7R)-7-(Tetrahydropyran-2-yloxy)-6-[(3S)-2-brom 3-(tetrahydropyran-2-yloxy)-4,4 trimethylen-non-1-en-7-inyl]-bicyclo [3.3.0] octan-3-on 105 mg der Titelverbindung als farbloses Öl.
IR : 3 600, 3 410 (breit), 2 938, 2 228, 1 710, 1 601, 1 010/cm

9 a) (1R,5S,6R,7R)-3,3-Äthylendioxy-7-benzoyloxy-6-(2-brom-3-oxo-4,4-trimethylen-non-1-en-7-inyl)-bicyclo [3.3.0] octan

In Analogie zu Beispiel 2 a erhält man aus 5 g (1R,5S,6R,7R)-3,3-Äthylendioxy-7-benzoyloxy-6-formyl-bicyclo [3.3.0] octan mit 2-Oxo-3,3-trimethylen-oct-6-in-phosphonsäuredimethylester und N-Bromsuccinimid 4,75g der Titelverbindung als farbloses Öl.
IR : 2 938, 2 876, 1 712, 1 688, 1 602, 1 588, 1 451, 1 270, 948/cm

9 b) (1R,5S,6R,7R)-7-Hydroxy-6-[(3S)-2-brom-3-hydroxy-4,4-trimethylen-non-1-en-7-inyl]-bicyclo [3.3.0] octan-3-on

In Analogie zu Beispiel 2 b erhält man aus 3,10 g des nach Beispiel 9 a hergestellten Ketons 700 mg der Titelverbindung (15α-Hydroxy) als farbloses Öl.
IR : 3 600, 3 410 (breit), 2 955, 2 930, 1 738, 1 600/cm

9 c) (1R,5S,6R,7R)-7-(tetrahydropyran-2-yloxy)-6-[(3S)-2-brom-3-(tetrahydropyran-2-yloxy)-4,4-trimethylen-non-1-en-7-inyl]-bicyclo [3.3.0] octan-3-on

In Analogie zu Beispiel 2 c erhält man aus 600 mg des nach Beispiel 9 b hergestellten Diols 730 mg der Titelverbindung als farbloses Öl.
IR : 2 940, 2 868, 1 740, 1 451, 1 120/cm

## Beispiel 10

(5E)-18,18,19,19-Tetradehydro-16,16-tetramethylen-6a-carbaprostaglandin-$I_2$

In Analogie zu Beispiel 1 erhält man aus 50 mg (1R,5S,6R,7R)-7(Tetrahydropyran-2-yloxy)-6-[(E)-(3R)-3-(tetrahydropyran-2-yloxy)-4,4-tetramethylen-oct-1-en-6-inyl]-bicyclo [3.3.0] octan-3-on 105 mg der Titelverbindung als farbloses Öl.
IR : 3 600, 3 525, 3 410 (breit), 2 938, 2 860, 1 710, 976/cm
Die Ausgangsmaterialien für die obige Titelverbindung werden wie folgt hergestellt :

10 a) 2-Oxo-3,3-tetramethylen-hept-5-in-phosphonsäure-dimethylester

Zu einer Lösung von 44,52 g Diisopropylamin in 200 ml Tetrahydrofuran gibt man bei − 30 °C 265 ml 1,66 M Butyllithium-Lösung in Hexan. Anschließend tropft man 22,83 g Cyclopentancarbonsäure zu, rührt 30 Minuten bei − 10 °C und tropft dann 31,92 g 1 Brom-2-butin zu, rührt 16 Stunden bei 25 °C und gießt auf 600 ml Eiswasser, säuert mit Salzsäure an, extrahiert mit Äther, wäscht den Extrakt mit Sole und engt im Vakuum ein. Nach Destillation des Rückstandes erhält man 21,30 g 2,2-Tetramethylen-hex-4-in-säure, Kp 155-157° bei 13 Torr.
IR : 3 520 (breit), 2 982, 2 960, 1 702, 1 425, 1 100, 1 035/cm
Zur Veresterung werden 20 g der Säure in 250 ml Methanol und 2,5 ml conc. Schwefelsäure 6

Stunden unter Rückfluß erhitzt. Man erhält 18,10 g 2,2-Tetramethylen-hex-4-in-säuremethylester, Kp 110-111 °C bei 14 Torr.

IR : 2 951, 2 860, 1 728, 1 430, 1 331, 1 098/cm

Dieser Ester wird gemäß Beispiel 1 a in die Titelverbindung überführt, Kp 120-122 °C bei 0,16 Torr.

IR : 3 425 (breit), 2 980, 2 951, 2 853, 1 705, 1 250, 1 033/cm

10 b) (1R,5S,6R,7R)-3,3-Äthylendioxy-7-benzoyloxy-6-[(E)-3-oxo-4,4-tetramethylen-oct-1-en-6-inyl]-bicyclo [3.3.0] octan

In Analogie zu Beispiel 1 b erhält man aus 5 g (1R,5S,6R,7R)-3,3-Äthylendioxy-7-benzoyloxy-6-formyl-bicyclo [3.3.0] octan mit 2-Oxo-3,3-tetramethylen-hept-5-in-phosphonsäuredimethylester 5,35 g der Titelverbindung als farbloses Öl.

IR : 2 940, 2 865, 1 711, 1 684, 1 622, 1 600, 1 585, 1 275, 978, 947/cm

10 c) (1R,5S,6R,7R)-3,3-Äthylendioxy-7-benzoyloxy-6[(E)-(3R)-3-hydroxy-4,4-tetramethylen-oct-1-en-6-inyl]-bicyclo [3.3.0] octan

In Analogie zu Beispiel 1 c erhält man aus 5,20 g des nach Beispiel 10 b hergestellten Ketons 2,95 g der Titelverbindung als farbloses Öl.

IR : 3 600, 3 515, 2 945, 2 878, 1 712, 1 601, 1 585, 1 274, 976, 947/cm

10 d) (1R,5S,6R,7R)-7-(Tetrahydropyran-2-yloxyl)-6-[(E)-(3R)-3-(tetrahydropyran-2-yloxy)-4,4-tetramethylenoct-1-en-6-inyl]-bicyclo [3.3.0] octan-3-on

In Analogie zu Beispiel 1 d erhält man aus 2,75 g der nach Beispiel 10 c hergestellten Verbindung 2,05 g der Titelverbindung als farbloses Öl.

IR : 2 943, 2 870, 1 740, 976/cm

## Beispiel 11

(5E)-18,19-Didehydro-19-methyl-16,16-trimethylen-6a-carba-prostaglandin-$I_2$

In Analogie zu Beispiel 1 erhält man aus 500 mg (1R,5S,6R,7R)-7-(Tetrahydropyran-2-yloxy)-6-[(E)-(3R)-7-methyl-3-(tetrahydropyran-2-yloxy)-4,4-trimethylenocta-1,6- dienyl]-bicyclo [3.3.0] octan-3-on 112 mg der Titelverbindung als farbloses Öl.

IR : 3 600, 3 520, 3 410 (breit), 2 935, 1 711, 1 601, 976/cm

Die Ausgangsmaterialien für die obige Titelverbindung werden wie folgt hergestellt :

11 a) 6-Methyl-2-oxo-3,3-trimethylen-hept-5-en-phosphonsäuredimethylester

Zu einer Lösung von 53,10 g Diisopropylamin in 250 ml Tetrahydrofuran gibt man bei − 30° 318 ml 1,66 M Butyllithiumlösung in Hexan und anschließend 24.02 g Cyclobutancarbonsäure. Man rührt noch 30 Minuten bei − 10 °C und tropft dann 42,91 g 1-Brom-3-methyl-2-buten zu und rührt 18 Stunden bei 25 °C. Anschließend gießt man auf 1 l Eiswasser, stellt mit 2N Salzsäure auf pH 4 ein, extrahiert mit Äther, wäscht den Extrakt mit Sole, trocknet über Magnesiumsulfat und dampft im Vakuum den Äther ab. Den Rückstand destilliert man und erhält 26,30 g 5-Methyl-2,2-trimethylen-hex-4-en-Säure (Kp 158-159 °C bei 14 Torr), die analog Beispiel 1 a 23,5 g 5-Methyl-2,2-trimethylen-hex-4-en-Säure-methylester (Kp 108-110 °C bei 14 Torr) liefert.

IR : 2 980, 2 948, 2 857, 1 728, 1 430, 1 080, 932/cm

Aus 20 g des vorstehend hergestellten Methylesters erhält man durch Umsetzung mit Methanphosphonsäuredimethylester gemäß Beispiel 1 a 23,30 g der Titelverbindung als farblose Flüssigkeit. Kp 138-140 °C bei 0,15 Torr.

IR : 3 420 (breit), 2 988, 2 950, 2 855, 1 704, 1 2450, 1 035/cm

11 b) (1R,5S,6R,7R)-3,3-Äthylendioxy-7-benzoyloxy-6-[(E)-7-methyl-3-oxo-4,4-trimethylen-octa-1,6-dienyl]-bicyclo [3.3.0] octan

In Analogie zu Beispiel 1 b erhält man aus 5 g (1R,5S,6R,7R)-3,3-Äthylendioxy-7-benzoyloxy-6-formyl-bicyclo [3.3.0] octan und dem nach Beispiel 11 a hergestellten Phosphonat 5,95 g der Titelverbindung als farbloses Öl.

IR : 2 948, 2 860, 1 712, 1 620, 1 600, 1 585, 1 272, 977, 948/cm

11 c) (1R,5S,6R,7R)-3,3-Äthylendioxy-7-benzoyloxy-6-[(E)-(3R)-3-hydroxy-7-methyl-4,4-trimethylen-octa-1,6-dienyl]-bicyclo [3.3.0] octan

In Analogie zu Beispiel 1 c erhält man aus 5,00 g des nach Beispiel 11 b hergestellten Ketons 2,88 g der Titelverbindung (15α-Hydroxy) als farbloses Öl.

IR : 3 600, 3 510, 2 940, 2 868, 1 712, 1 600, 1 584, 1 272, 978, 948/cm

11 d) (1R,5S,6R,7R)-7-(Tetrahydropyran-2-yloxy-6-[(E)-(3R)-7-methyl-3-(tetrahydropyran-2-yloxy)-4,4-trimethylen-octa-1,6-dienyl]-bicyclo [3.3.0] octan-3-on

In Analogie zu Beispiel 1 d erhält man aus 2,50 g der nach Beispiel 11 c hergestellten Verbindung 1,90 g der Titelverbindung als farbloses Öl.

IR : 2 940, 2 871, 1 738, 976/cm

Beispiel 12

(5E)-18,18,19,19-Tetradehydro-16,16-trimethylen-N-methansulfonyl-6a-carba-prostaglandin-$I_2$-carboxamid

Eine Lösung von 190 mg (5E)-18,18,19,19-Tetradehydro-16,16-trimethylen-6a-carba-prostaglandin-$I_2$ (hergestellt nach Beispiel 1) in 4 ml Dimethylformamid wird bei 0 °C mit 80 mg Chlorameisensäurebutylester und 60 mg Triäthylamin versetzt. Nach 30 minuten werden 240 mg des Natriumsalzes von Methylsulfonamid (hergestellt aus Methylsufonamid und Natriummethylat) und 1 ml Hexamethylphosphorsäuretriamid zugesetzt und 3 Stunden bei 25 °C gerührt. Anschließend gibt man das Reaktionsgemisch auf Citratpuffer (pH 5), extrahiert mehrere Male mit Ethylacetat, wäscht die organische Phase mit Sole, trocknet über Magnesiumsulfat und dampft im Vakuum ein. Nach Chromatographie des Rückstandes an Kieselgel mit Dichlormethan und 1-5 % Isopropanol erhält man 135 mg der Titelverbindung als Öl.

IR : 3 600, 3 410 (breit), 2 960, 2 855, 1 728, 976/cm

Beispiel 13

(5E)-13,14-Didehydro-18,18,19,19-tetradehydro-16,16-trimethylen-6a-carba-prostaglandin-$I_2$-carboxamid

200 mg (5E)-13,14-Didehydro-18,18,19,19-tetradehydro-16,16-trimethylen-6a-carba-prostanglandin-$I_2$ (s. Beispiel 2) werden in 5 ml Tetrahydrofuran gelöst und bei 0 °C mit 90 mg Triäthylamin und 98 mg Chlorameisensäureisobutylester versetzt. Nach 1 Stunde wird bei 0 °C für 10 Minuten Ammoniakgas eingeleitet, dann 1 Stunde bei 25° belassen. Man verdünnt dann mit Wasser, extrahiert mit Dichlormethan, wäscht den Extrakt mit Sole, trocknet über Magnesiumsulfat und dampft im Vakuum ein. Man reinigt durch Chromatographie an Kieselgel mit Chloroform/1-5 % Isopropanol und erhält 160 mg der Titelverbindung als farbloses Öl.

IR : 3 600, 3 540, 3 405, 2 955, 2 868, 2 220, 1 670, 1 588/cm

Beispiel 14

(5E)-18,19-Didehydro-19-chlor-16,16-trimethylen-6a-carbaprostaglandin-$I_2$

In Analogie zu Beispiel 1 erhält man aus 2 g (1R,5S,6R,7R)-7-(Tetrahydropyran-2-yloxy)-6-[(E)-(3R)-7-chlor-3-(tetrahydropyran-2-yloxy)-4,4-trimethylen-octa-1,6-dienyl]-bicyclo [3.3.0] octan-3-on durch Wittig-Reaktion (6 Stunden beim Raumtemperatur) und anschließende Schutzgruppenabspaltung 130 mg der Titelverbindung als farbloses Öl.

IR : (Film) : 3 500 (breit), 2 930, 2 850, 1 710, 1 660, 975/cm

Die Ausgangsmaterialien für die obige Titelverbindung werden wie folgt hergestellt :

14 a) 6-Chlor-2-oxo-3,3-trimethylen-hept-5-en-phosphonsäuredimethylester

Man gibt zu einer Lösung von 45 g Diisopropylamin in 200 ml Tetrahydrofuran bei − 30 °C 267 ml einer 1,66 M Butyllithiumlösung in Hexan und danach 20,2 g Cyclobutancarbonsäure. Nach 30-minütigen Nachrühren bei − 10 °C werden 30 g 1,3-Dichlor-buten-(2) zugetropft. Man läßt 16 Stunden bei Raumtemperatur rühren, gießt anschließend auf 1 l Eiswasser, stellt mit 2 N Salzsäure auf pH 4 ein, extrahiert mit Äther, wäscht die vereinigten organischen Phasen mit gesättigter Natriumchlorid-Lösung, trocknet sie über Magnesium-sulfat und engt im Vakuum ein. Der Rückstand wird durch Destillation gereinigt. Man erhält 22,3 g 5-Chlor-2,2-trimethylen-hex-4-ensäure (Kp 146-150 °C bei 4 Torr), die analog Beispiel 1 a 21 g 5-Chlor-2,2-trimethylen-hex-4-ensäure-methylester (Kp = 107-110 °C bei 4 Torr) liefern.

IR : (Film) : 2 970, 2 950, 2 890, 1 735, 1 665, 1 195, 1 170/cm

Gemäß der Vorschrift in Beispiel 1 a erhält man aus den 21 g Methylester durch Umsetzung mit Methanphosphonsäuredimethylester 20,5 g der Titelverbindung als farblose Flüssigkeit.

IR : (Film) : 3 400, 2 980, 2 945, 2 850, 1 710, 1 666, 1 260, 1 030/cm

14 b) (1R,5S,6R,7R)-3,3-Äthylendioxy-7-benzoyloxy-6-[(E)-7-chlor-3-oxo-4,4-trimethylen-octa-1,6-dienyl]-bicyclo [3.3.0] octan

In Analogie zur Vorschrift in Beispiel 1 b erhält man aus 6 g (1R,5S,6R,7R)-3,3-Äthylendioxy-7-benzoyloxy-6-formyl-bicyclo [3.3.0] octan und dem in Beispiel 14 a beschriebenen Phosphonat 6,2 g der Titelverbindung als farbloses Öl.

IR : (Film) : 2 950, 2 855, 1 720, 1 670, 1 630, 1 600, 1 583, 1 270, 715/cm

14 c) (1R,5S,6R,7R)-3,3-Äthylendioxy-7-benzoyloxy-6-[(E)-(3R)-7-chlor-3-hydroxy-4,4-trimethylen-octa-1,6-dienyl]-bicyclo [3.3.0] octan

In Analogie zu Beispiel 1 c erhält man aus 6,2 g des nach Beispiel 14 b hergestellten α,β-ungesättigten Ketons 3,2 g der Titelverbindung (15α-Alkohol) als farbloses Öl.

IR : (Film) : 3 500 (breit), 2 950, 2 860, 1 720, 1 665, 1 600, 1 580, 1 270, 715/cm

14 d) (1R,5S,6R,7R)-7-(Tetrahydropyran-2-yloxy-6-[(E)-(3R)-7-chlor-3-(tetrahydropyran-2-yloxy)-4,4-trimethylen-octa-1,6-dienyl]-bicyclo [3.3.0] octan-3-on

Analog zur Vorschrift in Beispiel 1 d erhält man aus 3,1 g der nach Beispiel 14 c hergestellten Verbindung 2,3 g der Titelverbindung als farbloses Öl.

IR (Film) : 2 940, 2 870, 1 735, 1 665, 1 180, 1 125, 1 080, 1 020/cm

## Beispiel 15

(5E)-3-Oxa-18,18,19,19-tetradehydro-16,16-trimethylen-6a-carbaprostaglandin-$I_2$

Zu einer Lösung von 510 mg 2-{(E)-(1S,5S,6R,7R,7-(Tetrahydropyran-2-yloxy)-6-[(E)-(3R)-3-(tetrahydropyran-2-yloxy)-4,4-trimethylenoct-1-en-6-inyl]-bicyclo [3.3.0] octan-3-yliden}-äthan-1-ol in 15 ml Tetrahydrofuran gibt man 113 mg 55 %ige Natriumhydridsuspension (in Mineralöl) und kocht 1 Stunde am Rückfluß. Man fügt eine Lösung von 166 mg Bromessigsäure in 5 ml Tetrahydrofuran zu, kocht 18 Stunden am Rückfluß, verdünnt mit Äther und schüttelt viermal mit je 25 ml 4 %iger Natronlauge. Dieser Extrakt wird mit 10 %iger Schwefelsäure bei 0 °C auf pH3 eingestellt und mit Methylenchlorid extrahiert. Der organische Extrakt wird mit Sole geschüttelt, über Magnesiumsulfat getrocknet und im Vakuum eingedampft. Man erhält 440 mg (5E)-3-Oxa-18,18,19,19-tetradehydro-16,16-trimethylen-6a-carba-prostaglandin-$I_2$-11,15-bis-(tetrahydropyranyläther), die man zur Abspaltung der Schutzgruppen 18 Stunden mit 30 ml Essigsäure/Wasser/Tetrahydrofuran bei 25 °C rührt. Man dampft unter Zusatz von Toluol ein und chromatographiert den Rückstand an Kieselgel mit Essigester/0,1-1 % Essigsäure. Dabei erhält man 283 mg der Titelverbindung als farbloses Öl.

IR($CHCl_3$) : 3 680, 3 400 (breit), 2 930, 1 730, 1 600, 1 425, 970/cm.

Das Ausgangsmaterial für die obige Titelverbindung wird wie folgt hergestellt :

15 a) 2-{(E)-(1S,5S,6R,7R)-7-(Tetrahydropyran-2-yloxy)-6-[(E)-(3R)-3-(tetrahydropyran-2-yloxy)-4,4-trimethylen-oct-1-en-6-inyl]-bicyclo [3.3.0] octan-3-yliden}-äthan-1-ol

Zu einer Lösung von 3,32 g Phosphonolessigsäuretriäthylester in 75 ml Tetrahydrofuran fügt man bei 0 °C 1,42 g Kalium-tert.-butylat, rührt 10 Minuten, versetzt mit einer Lösung aus 4 g (1R,5S,6R,7R)-7-(Tetrahydropyran-2-yloxy)-6-[(E)-(3R)-3-(tetrahydropyran-2-yloxy)-4,4-trimethylen-oct-1-en-6-inyl]-bicyclo [3.3.0] octan-3-on in 40 ml Toluol und rührt 18 Stunden bei 24 °C. Man verdünnt mit 400 ml Äther, schüttelt einmal mit Wasser, einmal mit 20 %iger Natronlauge, wäscht mit Wasser neutral, trocknet über Magnesiumsulfat und dampft in Vakuum ein. Den Rückstand filtriert man mit Hexan/Äther (1+1) über Kieselgel. Dabei erhält man 4 g des ungesättigten Esters als farbloses Öl.

IR : 2 940, 2 870, 1 700, 1 652, 972/cm

Man fügt 1,1 g Lithiumaluminiumhydrid portionsweise bei 0 °C zu einer gerührten Lösung von 4 g des vorstehend hergestellten Esters in 130 ml Äther und rührt 30 Minuten bei 0 °C. Man zerstört den Reagenzüberschuß durch tropfenweise Zugabe von Essigester, fügt 6 ml Wasser zu, rührt 2 Stunden bei 20 °C, filtriert und dampft im Vakuum ein. Den Rückstand chromatographiert man mit Äther/Hexan (3 + 2) an Kieselgel. Dabei erhält man als impolarere Verbindung 1,1 g 2-(Z)-(1S,5S,6R,7R)-7-(Tetrahydropyran-2-yloxy)-6-[(E)-(3R)-3-(tetrahydropyran-2-yloxy)-4,4-trimethylen-oct-1-en-6-inyl]-bicyclo [3.3.0] octan-3-yliden-äthan-1-ol und 2,1 g der Titelverbindung als farblose Öle.

IR : 3 600, 3 450, 2 940, 2 865, 1 600, 974/cm

## Beispiel 16

(5Z)-3-Oxa-18,18,19,19-tetradehydro-16,16-trimethylen-6a-carbaprostaglandin-$I_2$

In Analogie zu Beispiel 15 erhält man aus 490 mg 2-{(Z)-(1S,5S,6R,7R)-7-(Tetrahydropyran-2-yloxy-4,4-trimethylen-oct-1-en-6-inyl)-bicyclo [3.3.0] octan-3-yliden}-äthan-1-ol 220 mg der Titelverbindung als farbloses Öl.

IR : 3 600, 3 400 (breit), 2 930, 1 732, 1 600, 972/cm

Beispiel 17

(5E)-13,14-Didehydro-3-oxa-18,18,19,19-tetradehydro-16,16-trimethyl-en-6a-carba-prostaglandin-$I_1$

Zu einer Lösung von 260 mg 2-{(E)-(1S,5S,6S,7R)-7-(Tetrahydropyran-2-yloxy)-6-[(3S)-3-(tetrahydropyran-2-yloxy)-4,4-trimethylen-octa-1,6-diinyl]-bicyclo [3.3.0] octan-3-yliden}-äthan-1-ol in 7 ml Tetrahydrofuran gibt man 60 mg 55 %ige Natriumhydridsuspension (in Mineralöl) und kocht 1 Stunde am Rückfluß. Man fügt eine Lösung von 82 mg Bromessigsäure in 2,5 ml Tetrahydrofuran zu, kocht 18 Stunden am Rückfluß, verdünnt mit Äther und schüttelt viermal mit je 15 ml 4 %iger Natronlauge. Dieser Extrakt wird mit 10 %iger Schwefelsäure bei 0 °C auf pH3 eingestellt und mit Methylchlorid extrahiert. Der organische Extrakt wird mit Sole geschüttelt, über Magnesiumsulfat getrocknet und im Vakuum eingedampft. Man erhält 250 mg (5E)-13,14-Didehydro-3-oxa-18,18,19,19-tetradehydro-16,16-trimethyl-6a-carbaprostaglandin-$I_2$-11,15-bis-(tetrahydropyranyläther, die man zur Abspaltung der Schutzgruppen 18 Stunden mit 12 ml Essigsäure/Wasser/Tetrahydrofuran bei 25 °C rührt. Man dampft unter Zusatz von Toluol ein und chromatographiert den Rückstand an Kieselgel mit Essigester/0,1-1 % Essigsäure. Dabei erhält man 130 mg der Titelverbindung als farbloses Öl.

IR : 3 680, 3 400 (breit), 2 930, 2 225, 1 730, 1 602/cm.

Das Ausgangsmaterial für die obige Titelverbindung wird wie folgt hergestellt :

17 a) (1R,5S,6S,7R)-3,3-Äthylendioxy-7-(tetrahydropyran-2-yloxy)-6-[(3S)-2-brom-3-(tetrahydropyran-2-yloxy)-4,4-trimethylen-oct-1-en-6-inyl]-bicyclo [3.3.0] octan

Zu einer Lösung von 3 g (1R,5S,6S,7R)-3,3-Äthylendioxy-7-benzoyloxy-6-(2-brom-3-oxo-4,4-trimethylen-oct-1-en-6-inyl)-bicyclo [3.3.0] octan in 70 ml Methanol fügt man bei − 40 °C portionsweise 1,2 g Natriumborhydrid und rührt 45 Minuten bei − 40 °C. Anschliessend verdünnt man mit Äther, wäscht mit Wasser neutral, trocknet über Magnesiumsulfat und dampft im Vakuum ein.

Das Rohprodukt (15-Epimerengemisch) löst man in 100 ml Methanol, fügt 1,2 g Kaliumcarbonat zu und rührt 20 Stunden bei 23 °C unter Argon. Anschließend engt man im Vakuum ein, verdünnt mit Äther und wäscht mit Sole neutral. Man trocknet über Magnesiumsulfat und dampft im Vakuum ein. Durch Säulenchromatographie an Kieselgel mit Äther/Hexan (3 + 2) erhält man zunächst 0,8 g des 15β-konfigurierten Alkohols sowie als polarere Komponente 0,9 g der 15α-konfigurierten Verbindung (PG-Nomenklatur) als farblose Öl.

Eine Lösung aus 0,8 g des α-Alkohols, 8 mg p-Toluolsulfonsäure und 0,7 g Dihydropyran in 25 ml Methylenchlorid rührt man 45 Minuten bei 0 °C. Anschließend verdünnt man mit Äther, schüttelt mit verdünnter Natriumbicarbonatlösung, wäscht mit Wasser neutral, trocknet über Magnesiumsulfat und dampft im Vakuum ein. Nach Chromatographie des Rückstandes an Kieselgel erhält man mit Hexan/Äther (7 + 3) 1,1 g der Titelverbindung als farbloses Öl.

IR : 2 940, 2 875, 1 450, 948/cm

17 b) (1R,5S,6S,7R)-7-(Tetrahydropyran-2-yloxy)-6-[(3S)-3-(tetrahydropyran-2-yloxy)-4,4-trimethylen-octa-1,6-diinyl]-bicyclo [3.3.0] octan-3-on

Eine Lösung von 1,1 g der nach Beispiel 17a hergestellten Verbindung in 12 ml Dimethylsulfoxid und 5 ml Tetrahydrofuran gibt man 325 mg Kalium-tert.-butylat und rührt 2 Stunden bei 22 °C. Man verdünnt mit 50 ml Wasser und extrahiert dreimal mit je 50 ml Äther/Hexan (3 + 2), wäscht den Extrakt mit Wasser, trocknet über Magnesiumsulfat und dampft im Vakuum ein. Man rührt den Rückstand 20 Stunden mit 30 ml Essigsäure/Wasser/Tetrahydrofuran (65/35/10), dampft im Vakuum ein und reinigt den Rückstand durch Chromatographie an Kieselgel. Mit Äther eluiert man 0,6 g ölige Substanz, die man in 20 ml Methylenchlorid mit 0,45 g Dihydropyran und 5 mg p-Toluolsulfonsäure bei 0 °C umsetzt. Nach 30 Minuten verdünnt man mit Äther, schüttelt mit Natriumhydrogencarbonatlösung und Wasser, trocknet über Magnesiumsulfat und dampft im Vakuum ein. Nach Chromatographie an Kieselgel mit Hexan/Äther (1 + 1) erhält man 0,8 g der Titelverbindung als farbloses Öl.

IR : 2 945, 2 880, 2 210, 1 736/cm.

17 c) 2-{(E)-(1S,5S,6S,7R)-7-(Tetrahydropyran-2-yloxy)-6-[(3S)-3-(tetrahydropyran-2-yloxy)-4,4-trimethylen-octa-1,6-diinyl]-bicyclo [3.3.0] octan-3-yliden}-Äthan-1-ol

Zu einer Lösung von 0,66 g Phosphonoessigsäuretriäthylester in 15 ml Tetrahydrofuran fügt man bei 0 °C 280 mg Kalium-tert.-butylat, rührt 10 Minuten, versetzt mit einer Lösung aus 0,8 g des nach Beispiel 17 b hergestellten Ketons in 8 ml Toluol und rührt 18 Stunden bei 24 °C. Man verdünnt mit 100 ml Äther, schüttelt einmal mit Wasser, einmal mit 20 %iger Natronlauge, wäscht mit Wasser neutral, trocknet über

Magnesiumsulfat und dampft im Vakuum ein. Den Rückstand filtriert man mit Hexan/Äther (1 + 1) über Kieselgel. Dabei erhält man 0,81 g des ungesättigten Esters als farbloses Öl.
IR : 2 942, 2 873, 2 210, 1 700, 1 650/cm

Man fügt 220 mg Lithiumaluminiumhydrid portionsweise bei 0 °C zu einer gerührten Lösung von 0,8 g des vorstehend hergestellten Esters in 25 ml Äther und rührt 30 Minuten bei 0 °C. Man zerstört den Reagenzüberschuß durch tropfenweise Zugabe von Essigester, fügt 1 ml Wasser zu, rührt 2 Stunden bei 20 °C, filtriert und dampft im Vakuum ein. Den Rückstand chromatographiert man mit Äther/Hexan (3 + 2) an Kieselgel. Dabei erhält man als impolarere Verbindung 230 mg 2-{(Z)-(1S,5S,6S,7R)-7-(Tetrahydropyran-2-yloxy)-6-[(3S)-3-(tetrahydropyran-2-yloxy)-4,4-trimethylen-octa-1,6-diinyl]-bicyclo [3.3.0] octan-3-yliden}-äthan-1-ol und 240 mg der Titelverbindung als farblose Öle.
IR : 3 600, 3 450, 2 940, 2 215/cm.

Beispiel 18

(5E)-13,14-Didehydro-20-methyl-3-oxa-18,18,19,19-tetradehydro-16,16-trimethylen-6a-carba-prostaglandin-$I_2$

In Analogie zu Beispiel 15 erhält man aus 200 mg 2-(E)-(1S,5S,6R,7R)-7-(Tetrahydropyran-2-yloxy)-6-[(3S)-3-(tetrahydropyran-2-yloxy)-4,4-trimethylen-nona-1,6-diinyl]-bicyclo [3.3.0] octan-3-yliden-äthan-1-ol 89 mg der Titelverbindung als farbloses Öl.
IR : 3 600, 3 406 (breit), 2 930, 2 862, 2 221, 1 730, 1 600/cm.

18 a) 2-{(E)-(1S,5S,6S,7R)-7-(Tetrahydropyran-2-yloxy)-6-[(3S)-3-(tetrahydropyran-2-yloxy)-4,4-trimethylen-nona-1,6-diinyl]-bicyclo [3.3.0] octan-3-yliden}-äthan-1-ol

In Analogie zu Beispiel 15 a erhält man aus 1,50 g (1R,5S,6R,7R)-7-(Tetrahydropyran-2-yloxy)-6-[(3S)-3-(tetrahydropyran-2-yloxy)-4,4-trimethylen-nona-1,6-diinyl]-bicyclo [3.3.0] octan-3-on 420 mg 2-{(Z)-(1S,5S,6R,7R)-7-(Tetrahydropyran-2-yloxy)-6-[(3S)-3-(tetrahydropyran-2-yloxy)-4,4-trimethylen-nona-1,6-diinyl]-bicyclo [3.3.0] octan-3-yliden}-äthan-1-ol als impolarere Verbindung. Als polarere Verbindung erhält man 820 mg der Titelverbindung als farbloses Öl.
IR : 3 600, 3 420, 2 948, 2 862, 2 225, 1 600/cm

Beispiel 19

(5E)-18,19-Didehydro-19-methyl-3-oxa-16,16-trimethylen-6a-carbaprostaglandin-$I_2$

In Analogie zu Beispiel 15 erhält man aus 250 mg 2-{(E)-(1S,5S,6R,7R)-7-(Tetrahydropyran-2-yloxy)-6-[(E)-(3R)-3-(tetrahydropyran-2-yloxy)-7-methyl-4,4-trimethylen-octa-1,6-dienyl]-bicyclo [3.3.0] octan-3-yliden}-äthan-1-ol 110 mg der Titelverbindung als farbloses Öl.
IR : 3 600, 3 415 (breit), 2 942, 2 860, 1 734, 1 601, 976/cm

19 a) 2-{(E)-(1S,5S,6R,7R)-7-(Tetrahydropyran-2-yloxy)-6-{(E)-(3R)-3-(tetrahydropyran-2-yloxy)-7-methyl-4,4-trimethylen-octa-1,6-dienyl]-bicyclo [3.3.0] octan-3-yliden}-äthan-1-ol

In Analogie zu Beispiel 15a erhält man aus 3 g (1R,5S,6R,7R)-7-(tetrahydropyran-2-yloxy)-6-[(E)-(3R)-7-methyl-3-(tetrahydropyran-2-yloxy)-4,4-trimethylen-octa-1,6-dienyl] bicyclo [3.3.0] octan-3-on als polarere Verbindung 1,60 g der Titelverbindung als farbloses Öl.
IR : 3 600, 3 410, 2 938, 2 858, 1 160, 1 015, 974/cm

Beispiel 20

(5E)-13,14-Didehydro-20-methyl-3-oxa-18,18,19,19-tetradehydro-16,16-trimethylen-6a-carba-prostaglandin-$I_2$-methylester

Zu einer Lösung von 100 mg (5E)-13,14-Didehydro-20-methyl-3-oxa-18,18,19,19-tetradehydro-16,16-trimethylen-6a-carba-prostaglandin-$I_2$ (siehe Beispiel 5) in 20 ml Dichlormethan + 5 ml Dimethoxyäthan tropft man bei 0 °C solange eine ätherische Diazomethanlösung bis die Gelbfärbung der Lösung bestehen bleibt. Man dampft im Vakuum ein und reinigt den Rückstand durch Chromatographie an Kieselgel mit Dichlormethan/2 % Isopropylalkohol und erhält 80 mg der Titelverbindung als farbloses Öl.
IR : 3 600, 2 942, 2 860, 2 228, 1 742, 1 135/cm

Beispiel 21

(5E)-3-Oxa-18,18,19,19-tetradehydro-16,16-trimethylen-6a-carbaprostaglandin-$I_2$-acetylamid

Zu einer Lösung von 255 mg (5E)-3-oxa-18,18,19,19-tetradehydro-16,16-trimethylen-6a-carba-prostaglandin-$I_2$-11,15-bis-(tetrahydropyranyläther) (siehe Beispiel 15) in 5 ml Acetonitril gibt man 63 mg Triäthylamin und anschließend bei 0 °C eine Lösung von 48 mg Acetylisocyanat in 2 ml Acetonitril. Nach 2 Stunden bei 20 °C engt man im Vakuum ein, verdünnt mit 100 ml Citratpuffer (pH5), extrahiert mit Dichlormethan, wächst der Extrakt nacheinander mit Natriumhydrogenkarbonatlösung und Sole, trocknet über Magnesiumsulfat und dampft im Vakuum ein. Zur Abspaltung der Schutzgruppen rührt man den Rückstand mit 10 ml Eisessig/Wasser/Tetrahydrofuran (65/35/10) 18 Stunden und dampft im Vakuum ein. Den Rückstand chromatographiert man an Kieselgel mit Dichlormethan/3 % Isopropylalkohol. Dabei erhält man 105 mg der Titelverbindung als farbloses Öl.

IR : 3 600, 3 500, 3 410, 2 950, 2 862, 1 725, 1 138, 976/cm

Beispiel 22

(5E)-3-Oxa-20-methyl-18,18,19,19-tetradehydro-16,16-trimethylen-6a-carba-prostaglandin-$I_2$-carboxamid

Zu einer Lösung von 196 mg (5E)-3-Oxa-20-methyl-18,18,19,19-tetradehydro-16,16-trimethylen-6a-carba-prostaglandin-$I_2$ in 5 ml Dimethylformamid gibt man bei 0 °C 65 mg Triäthylamin und 90 mg Chlorameisensäureisobutylester. Nach einer Stunde leitet man für 15 Minuten trockenes Ammoniakgas und die Lösung und läßt dann 2 Stunden bei 0 °C stehen. Zum Aufarbeiten verdünnt man mit Citratpuffer (pH5), extrahiert mit Äthylacetat, wäscht mit Natriumhydrogenkarbonatlösung und Sole, trocknet über Magnesiumsulfat und dampft im Vakuum ein. Nach Chromatographie des Rückstands an Kieselgel mit Dichlormethan/2 % Isopropanol erhält man 150 mg der Titelverbindung als farbloses Öl.

IR : 3 600, 3 495, 3 410, 2 955, 2 868, 1 662, 1 135, 976/cm

Beispiel 23

(5E)-13,14-Didehydro-20-methyl-3-oxa-18,18,19,19-tetradehydro-16,16-trimethylen-6a-carba-prostaglandin-$I_2$ (2,3-dihydroxy-propyl)-amid

Zu einer Lösung von 192 mg (5E)-13,14-Didehydro-20-methyl-3-oxa-18,18,19,19-tetradehydro-16,16-trimethylen-6a-carba-prostaglandin-$I_2$ in 4 ml Aceton gibt man bei 0 °C 59 mg Triäthylamin und 80 mg Chlorameisensäureisobutylester. Nach 30 Minuten versetzt man mit 240 mg 1-Amino-2,3-dihydroxypropan, 3 ml Aceton und 5 ml Acetonitril, hält 2 Stunden bei 20 °C, engt im Vakuum ein, verdünnt mit 100 ml Dichlormethan, schüttelt mit Sole, trocknet über Magnesiumsulfat und dampft im Vakuum ein. Nach Chromatographie des Rückstandes an Kieselgel mit Methylenchlorid/20 % Isopropylalkohol erhält man 150 mg der Titelverbindung als farbloses Öl.

IR : 3 600, 3 420 (breit), 2 952, 2 865, 2 221, 1 653, 1 135, 1 030/cm

Beispiel 24

(5E)-13,14-Didehydro-20-methyl-3-oxa-18,18,19,19-tetradehydro-16,16-trimethylen-6a-carba-prostaglandin-$I_2$-phenacylester

Man löst 200 mg (5E)-13,14-Didehydro-20-methyl-3-oxa-18,18,19,19-tetradehydro-16,16-trimethylen-6a-carba-prostaglandin-$I_2$ in 8 ml Aceton, versetzt mit 150 mg ω-Bromacetophenon und 2 ml Triäthylamin und rührt über Nacht bei Raumtemperatur. Man versetzt mit 200 ml Äther, schüttelt nacheinander mit Wasser und Sole, trocknet über Magnesiumsulfat und dampft im Vakuum ein. Den Rückstand reinigt man durch Chromatographie an Kieselgel mit Dichlormethan/2 % Aceton und erhält 190 mg der Titelverbindung.

IR : 3 600, 2 950, 2 866, 2 225, 1 748, 1 138, 1 028/cm

Beispiel 25

(5E)-2-Descarboxy-13,14-didehydro-20-methyl-3-oxa-2-(2-oxazolin-2-yl)-18,18,19,19-tetradehydro-16,16-trimethylen-6a-carba-prostaglandin-$I_2$

180 mg (5E)-13,14-Didehydro-20-methyl-3-oxa-18,18,19,19-tetradehydro-16,16-trimethylen-6a-carba-prostaglandin-$I_2$ löst man in 5 ml Hexamethyldisilazan und erhitzt 90 Minuten auf 140 °C, dann destilliert man den Reagenzüberschuß im Vakuum ab. Den Rückstand löst man in 5 ml Acetonitril, versetzt mit 786 mg Triphenylphosphin und 1 ml Triäthylamin und tropft bei 0 °C, 0,5 ml einer 1 M Lösung von Äthanolamin in Acetonitril zu. Nach 18 Stunden bei 20 °C engt man im Vakuum ein und wäscht den Rückstand fünfmal mit je 75 ml Hexan. Die gebildeten Kristalle werden vom öligen Rückstand abgetrennt und der ölige Rückstand in 15 ml Methanol gelöst, bei 0 °C mit 5 ml 2 N Natronlauge versetzt und 3 C Minuten bei 20 °C gerührt. Nach dem Einengen im Vakuum auf ca. 5 ml versetzt man mit 10 ml Wasser

und extrahiert viermal mit je 10 ml Äthylacetat. Nach Trocknen über Magnesiumsulfat und Eindampfen erhält man einen öligen Rückstand, den man an Kieselgel mit Dichlormethan/2 % Isopropylalkohol chromatographiert. Man erhält 100 mg der Titelverbindung als Öl.

Beispiel 26

(5E)-13,14-Didehydro-20-methyl-3-oxa-18,18,19,19-tetradehydro-16,16-trimethylen-6a-carba-prostaglandin-$I_2$-tris-(hydroxymethyl)-aminomethan-salz

Zu einer Lösung von 168 mg (5E)-13,14-Didehydro-20-methyl-3-oxa-18,18,19,19-tetradehydro-16,16-trimethylen-6a-carba-prostaglandin-$I_2$ in 34 ml Acetonitril fügt man bei 70 °C eine Lösung von 60 mg Tris-(hydroxymethyl)-aminomethan in 0,2 ml Wasser. Man läßt unter Rühren abkühlen, dekantiert nach 16 Stunden vom Lösungsmittel und trocknet den Rückstand im Vakuum. Man isoliert 145 mg der Titelverbindung als wachsartige Masse.

**Patentansprüche**

1. Carbacyclinderivate der allgemeinen Formel I

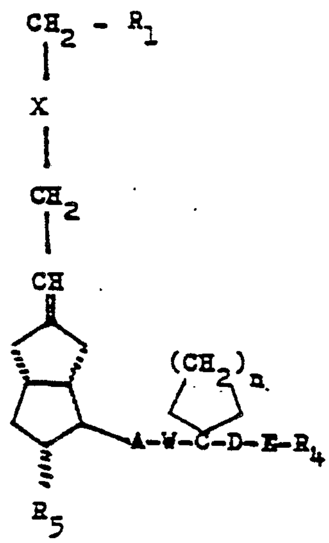

(I)

worin

$R_1$ den Rest $CH_2OH$ oder

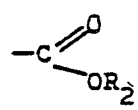

mit $R_2$ in der Bedeutung eines Wasserstoffatoms, eines Alkyl-, Cycloalkyl, Aryl, eines

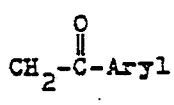

oder heterocyclischen Restes oder

$R_1$ den Rest

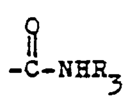

mit $R_3$ in der Bedeutung eines Alkanoyl- oder Alkansulfonylrestes mit je 1-10 C-Atomen oder des Restes $R_2$ oder

$R_1$ den Rest

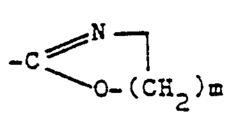

worin m die Zahl 1 oder 2 bedeutet,

X ein Sauerstoffatom oder eine $CH_2$-Gruppe,

A eine trans —CH = CH- oder —C ≡C-Gruppe,

W eine freie oder funktionell abgewandelte Hydroxymethylengruppe, wobei die OH-Gruppe α- oder β-ständig sein kann,

n die Zahl 1, 2 oder 3 bedeuten kann,

D eine geradkettige Alkylengruppe mit 1-3-C-Atomen,

E eine —C ≡ C-Bindung oder eine —$CR_6$ ≡ $CR_7$-Gruppe darstellt, wobei $R_6$ und $R_7$ sich unterscheiden und ein Wasserstoffatom oder eine Alkylgruppe mit 1-5 C-Atomen bedeuten oder ein Wasserstoffatom oder ein Halogenatom, vorzugsweise Chlor bedeuten,

$R_4$ eine Alkyl-, Cycloalkyl- oder eine gegebenenfalls substituierte Arylgruppe oder eine heterocyclische Gruppe,

$R_5$ eine freie oder funktionell abgewandelte Hydroxygruppe, und, falls $R_2$ die Bedeutung eines Wasserstoffatoms hat, deren Salze mit physiologisch verträglichen Basen bedeuten.

2. Verfahren zur Herstellung von Carbacyclinderivaten der allgemeinen Formel I, dadurch gekennzeichnet, daß man

a) eine Verbindung der allgemeinen Formel II,

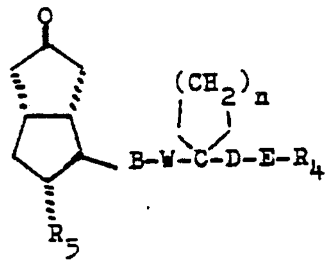

(II)

worin $R_5$, W, n, D, E und $R_4$ die obenangegebenen Bedeutungen aufweisen und B eine trans-Doppelbindung oder eine —CH = CBr-Gruppe bedeutet, gegebenenfalls nach Schutz anwesender freier Hydroxygruppen mit einem Wittig-Reagenz der Formel III

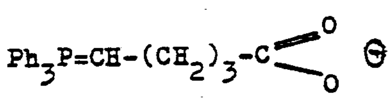

(III)

umsetzt oder

b) eine Verbindung der allgemeinen Formel IV,

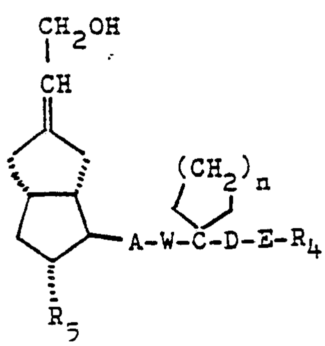

(IV)

worin $R_4$, $R_5$, A, W, D, E, n, die obenangegebenen Bedeutungen aufweisen, gegebenenfalls nach Schutz anwesender freier Hydroxygruppen mit einem Halogenessigsäurederivat der allgemeinen Formel V,

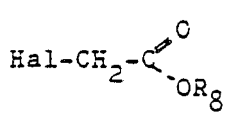

(V)

wobei Hal ein Chlor- Brom- oder Jodatom und $R_8$ einen Alkylrest oder Trialkyl-silylrest mit $C_1$-$C_4$-Alkylgruppen oder ein Alkalimetall (Na, Li, K) bedeutet, in Gegenwart einer Base veräthert und in den nach a) oder b) erhaltenen Verfahrensprodukten gegebenenfalls anschließend in beliebiger Reihenfolge Isomere trennt und/oder geschützte Hydroxygruppen freisetzt und/oder freie Hydroxygruppen verestert oder veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

3. Arzneimittel, bestehend aus einer oder mehreren Verbindungen des Anspruchs 1 und üblichen Hilfs- und Trägerstoffen.

**Claims**

1. Carbacyclin derivatives of general formula I

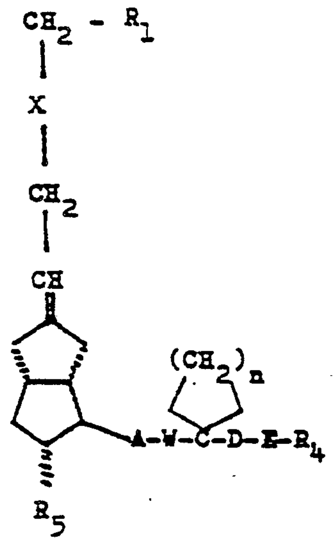

(I)

wherein

$R_1$ is $CH_2OH$ or

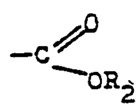

in which $R_2$ is hydrogen, alkyl, cycloalkyl, aryl, a

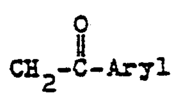

group or a heterocyclic group or $R_1$ is

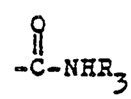

in which $R_3$ is alkanoyl or alkanesulfonyl of 1-10 carbon atoms or is the group $R_2$ or $R_1$ is

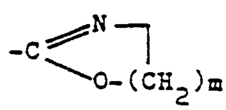

in which m is 1 or 2,

X is oxygen or a $CH_2$ group,

A is a trans —CH = CH- or —C ≡ C-group,

W is a free of functionally modified hydroxymethylene group in which the OH is in the α or β configuration,

n is 1, 2 or 3,

D is straight chain alkylene group of 1-3 carbon atoms,

E is a —C = C- bond or a —$CR_6$ = $CR_7$ group, in which $R_6$ and $R_7$ are different and one is hydrogen and the other is an alkyl group of 1-5 carbon atoms or halogen, preferably chlorine,

$R_4$ is alkyl, cycloalkyl, optionally substituted aryl or a heterocyclic group, and $R_5$ is free or functionally modified hydroxy group, and when $R_2$ is hydrogen, salts of these compounds with physiologically acceptable bases.

2. Process for the preparation of carbacyclin derivatives of the general formula I characterised in that

a) a compound of general formula II

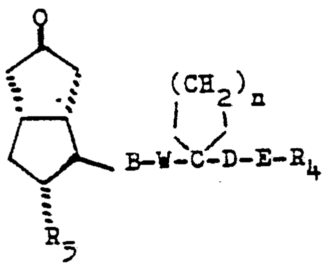

(II)

in which $R_5$, W, n, D, E and $R_4$ have the meanings given above and B is a trans double bond or a —CH = CHBr group, is reacted after optional protection of any free hydroxy groups present, with a Wittig reagent of formula III

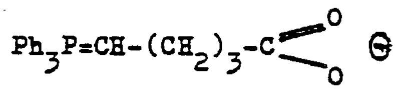

(III)

or

b) a compound of general formula IV

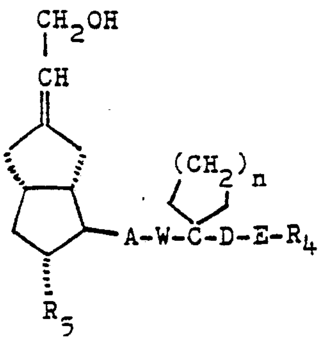

(IV)

wherein $R_4$, $R_5$, A, W, D, E and n have the meanings given above, is reacted, after optional protection of any free hydroxy groups present, with a haloacetic acid derivative of general formula V

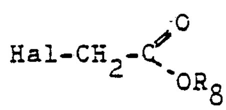

(V)

in which Hal is chlorine, bromine or iodine and $R_8$ is an alkyl or trialkylsilyl group, in which the alkyl groups are $C_{1-4}$, or an alkali metal (Na, Li or K), and the products resulting from (a) or (b) are optionally, and in an arbitrary sequence, separated into isomers and/or protected hydroxy groups are freed and/or free hydroxy groups are esterified or etherified and/or free carboxyl groups are esterified and/or esterified carboxyl groups are saponified or a carboxyl groups is convered to an amide or into a salt with a physiologically acceptable base.

3. A pharmaceutical composition comprising one or more compounds of claim 1 and conventional diluents or carriers.

**Revendications**

1. Carbacyclines répondant à la formule générale 1 :

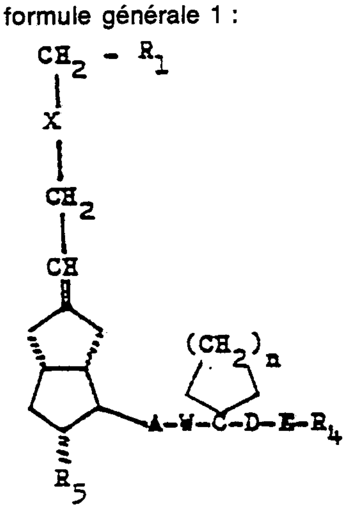

(I)

dans laquelle :

$R_1$ représente un radical $—CH_2OH$ ou un radical

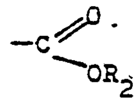

dans lequel $R_2$ désigne un atome d'hydrogène, un radical alkyle, cycloalkyle, aryle ou aroylméthyle

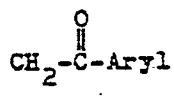

ou un radical hétérocyclique, ou

$R_1$ représente un radical

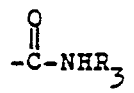

dans lequel $R_3$ désigne un radical alcanoyle ou un radical alcane-sulfonyle contenant chacun de 1 à 10 atomes de carbone ou désigne un radical $R_2$, ou

$R_2$ représente un radical

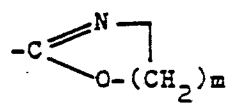

dans lequel m est égal à 1 ou à 2,

X représente un atome d'oxygène ou un radical $—CH_2—$,

A représente un radical —CH = —CH— trans ou un radical —C ≡ C—,

W représente un radical hydroxyméthylène libre ou sous la forme d'un dérivé fonctionnel, le radical hydroxy ayant la configuration α ou la configuration β,

n représente l'un des nombres 1, 2 et 3,

D représente un radical alkylène linéaire contenant de 1 à 3 atomes de carbone,

E représente un radical —C ≡ C— ou un radical $—CR_6 = CR_7—$ dans lequel $R_6$ et $R_7$ sont différents l'un de l'autre et représentent chacun un atome d'hydrogène ou un radical alkyle contenant de 1 à 5 atomes de carbone ou représentent chacun un atome d'hydrogène ou un atome d'halogène, de préférence de chlore,

$R_4$ représente un radical alkyle, un radical cycloalkyle, un radical aryle éventuellement substitué ou un radical hétérocyclique, et

$R_5$ représente un radical hydroxy libre ou sous la forme d'un dérivé fonctionnel,

et également, dans le cas où $R_2$ désigne un atome d'hydrogène, les sels que forment ces composés avec des bases acceptables du point de vue physiologique.

2. Procédé de préparation de carbacyclines de formule générale I, procédé caractérisé en ce que :

a) on fait réagir un composé répondant à la formule générale II :

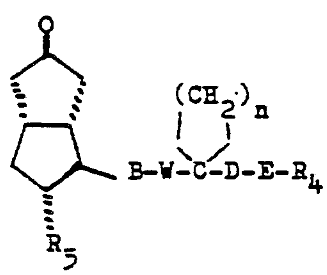

(II)

dans laquelle $R_5$, W, n, D, E et $R_4$ ont les significations précédemment données et B représente une double liaison trans ou un radical —CH ≡ CBr—, éventuellement après avoir protégé d'éventuels radicaux hydroxy, avec un réactif de Wittig de formule III

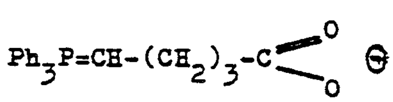

(III)

ou

b) on éthérifie, en présence d'une base, un composé répondant à la formule générale IV :

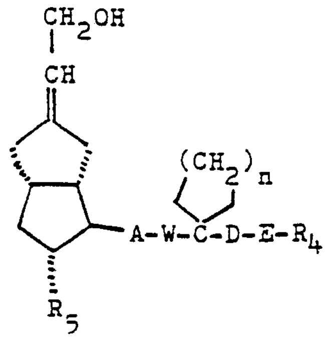

(IV)

dans laquelle $R_4$, $R_5$, A, W, D, E et n ont les significations précédemment données, éventuellement après avoir protégé d'éventuels radicaux hydroxy libres, avec un dérivé d'acide halogéno-acétique répondant à la formule générale V :

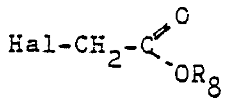

(V)

dans laquelle Hal représente un atome de chlore, de brome ou d'iode et $R_8$ représente un radical alkyle ou radical trialkylsilyle contenant de 1 à 4 atomes de carbone dans chacun des alkyles ou représente un métal alcalin (Na, Li, K), et ensuite, sur les produits réactionnels obtenus selon a) ou b), on effectue éventuellement, dans l'ordre que l'on veut, les opérations suivantes : on sépare des isomères et/ou on libère des radicaux hydroxy protégés et/ou on estérifie ou éthérifie des radicaux hydroxy libres et/ou on estérifie un radical carboxy libre et/ou on saponifie un radical carboxy estérifié ou on transforme un radical carboxy en un amide ou, avec une base acceptable du point de vue physiologique, en un sel.

3. Médicament constitué d'un ou plusieurs composés selon la revendication 1 ainsi que d'adjuvants et d'excipients usuels.